# EUROPEAN PATENT APPLICATION

(11) **EP 4 800 643 A1**
(43) Date of publication of application: **02.09.2026**
(21) Application number: 26161444.0
(22) Date of filing: 27.02.2026
(51) Int. Cl.: G06T 7/00, A61B 6/50, A61B 6/00

(54) **METHOD AND SYSTEM FOR ARTIFICIAL INTELLIGENCE BASED BONE MINERAL DENSITY ASSESSMENT FROM COMPUTER TOMOGRAPHY SCANS**

(30) Priority: 28.02.2025 US 202563764573 P; 30.01.2026 US 202663972741 P
(71) Applicant: Westerhoff, Malte, 12163 Berlin (DE); Lindow, Norbert, 12163 Berlin (DE); Herter, Felix, 12163 Berlin (DE); Bousabarah, Khaled, 12163 Berlin (DE); Visage Imaging GmbH, 12163 Berlin (DE)
(72) Inventor: Westerhoff, Malte, 12163 Berlin (DE); Lindow, Norbert, 12163 Berlin (DE); Herter, Felix, 12163 Berlin (DE); Bousabarah, Khaled, 12163 Berlin (DE)
(74) Representative: Schiweck Weinzierl Koch Patentanwälte Partnerschaft mbB

(57) **Abstract**

In an embodiment of the present invention, a method converts continuous scores from an AI disease classifier into clinically meaningful risk estimates. Historical score-outcome data are binned into percentiles, and the cumulative disease fraction is fitted with a differentiable curve for example a two-Beta mixture. The curve's derivative yields an instantaneous risk ratio that, combined with baseline prevalence, provides an absolute disease malady probability. Risk ratio and absolute probability ensures comparability across model versions, improving interpretability. The invention includes associated systems, software, and computer-readable media.

## Description

### FIELD OF INVENTION

The invention is in the field of medical image analysis, osteoporosis screening, and computer-aided diagnosis.

### BACKGROUND OF THE INVENTION

In order to diagnose a traditional X-Ray examination, the images printed on films would be 'hung' in front of a light box. For multi-image examinations, as well as for comparison with priors, the 'hanging' would often follow a specific protocol. For example, a particular organization or doctor may choose for a two-view chest X-Ray with a two-view prior exam, that the films be hung from left to right as follows: Frontal view of current examination, lateral view of current examination, frontal view of prior examination, lateral view of prior examination. In contrast, the doctor may hang mammography exams with the corresponding views of current and prior next to each other, if that was more appropriate for the diagnostic workflow in that case. Thus, the organization or doctor developed a traditional 'Hanging Protocol'. Currently, the film and the light box are often being replaced by computer systems, called PACS (Picture Archiving and Communication System). PACS systems can mimic the Hanging Protocols.

Traditional X-Ray examinations typically produce one or a small number of single two-dimensional (2D) images. In contrast, the more advanced imaging modalities such as Computer Tomography (CT) produce dozens of series of x-rays, each consisting of a hundred or more images. With the rapid increase in the amount of information that can be acquired using imaging technology, a number of problems arise for a diagnostician, for example a physician. Namely, integration, and display of relevant information is extremely difficult for a person to carry out without sophisticated computer processing. Embodiments of this invention therefore provide a computer-based analytic framework whereby image-based information can provide relevant information, e.g., to display information for a physician to diagnose and evaluate a patient's condition. The outcome can be serious, e.g., misdiagnoses of a patient can result in increased chance of morbidity and/or mortality.

### OBJECTIVE OF THE INVENTION

It is an object of the present invention to overcome or ameliorate at least one of the problems or disadvantages of the prior art. Any objects of the invention referred to herein or taken from this description should be read disjunctively and with the alternative object of to at least provide the public with a useful choice.

### SUMMARY OF THE INVENTION

The invention provides a computer-implemented system and method for automatically calculating Bone Mineral Density (BMD) in the trabecular (spongy) bone of vertebral bodies from routine 3D Computed Tomography (CT) scans. The system uses Artificial Intelligence (AI) and advanced image processing to identify vertebrae in 3D CT images and measure their trabecular attenuation (in Hounsfield Units, HU) as a surrogate for BMD. Key innovative steps include:

### (A) Automated Segmentation and Labeling

In an embodiment of the present invention, a trained Convolutional Neural Network (CNN) segments the 3D CT scan, identifying and labeling each thoracic and lumbar vertebral body in the scan volume. This segmentation isolates the vertebral trabecular bone for analysis.

### (B) Consistency and Quality Checks

In an embodiment of the present invention, the system performs checks to ensure the segmentation results are anatomically valid. For example: (i) each identified vertebral body forms one contiguous 3D connected component (except in cases where a vertebra is cut off at the scan boundary), and (ii) the vertebrae are in the correct anatomical order. The ordering check may use geometric criteria such as the relative positions, distances, and angles between the centers of mass of consecutive vertebrae to verify that labels increment consistently from cervical/thoracic to lumbar spine. These safeguards detect segmentation errors or mislabeling and allow the system to reject or correct invalid analyses;

### (C) Volumetric Region Of Interest (ROI) Placement for BMD Measurement

In an embodiment of the present invention, for each properly segmented vertebra, the system calculates the vertebra's volume and identifies an internal ROI for measuring trabecular attenuation. The ROI is a three-dimensional spherical volume whose size is a fixed percentage of the vertebra's total volume (for example, 5% of the vertebral body volume). The ROI is automatically placed entirely *inside* the trabecular portion of the vertebra, away from cortical bone boundaries. Critically, the placement algorithm avoids the posterior central region of the vertebral body where the BF and surrounding vascular channels are located (to prevent including the low-density venous plexus or sclerosis around these entry points in the measurement). The system preferentially positions the spherical ROI in an area of relatively low attenuation (lower bone mineral density) within the vertebra's trabecular bone. By using a volumetric ROI (covering many voxels in 3D) instead of a single 2D slice patch, the method reduces the influence of local inhomogeneities and yields a more reliable measure of overall trabecular density;

In an embodiment of the present invention, the ROI's center is chosen such that: (i) the sphere lies completely inside the vertebral body segmentation, (ii) the mean density of bone within the sphere is the lowest obtainable while satisfying (i) (this finds the most osteopenic region of the trabeculae), and (iii) the sphere is located anterior to the BF (thus excluding the posterior vascular channel region). Once placed, the median HU value of all voxels inside this spherical ROI is computed as the representative attenuation for that vertebra. Using the median rather than the mean makes the measurement more robust against outlier voxels (for instance, any small sclerotic spots or artifacts).

### (D) Calibration of Measurements Across Scanner Settings

In an embodiment of the present invention, the system calibrates the extracted HU values to account for differences in scanner models and scanning parameters (in particular, the X-ray tube voltage, or kVp). In CT imaging, the same physical bone can yield different HU values under different tube voltage settings or on different scanner manufacturers/models. In an embodiment of the present invention, the one or more acquisition parameters can be selected from the group consisting of a model of a three-dimensional Computer Tomography scanner used to generate the three-Dimensional Computer Tomography Image and an X-ray tube voltage used to generate the three-Dimensional Computer Tomography Image.

In various embodiments of the present invention, the above variations delineated in (A) - (D) can lead to inconsistency in BMD estimates and potentially misclassification of osteoporosis risk.

In an embodiment of the present invention, to overcome the above variations delineated in (A) - (D), the invention uses a calibration mapping to convert each measured ROI median value to an equivalent value under a reference standard condition (for example, a 120 kVp scan on a reference scanner model). In one embodiment, a linear mapping factor is applied: *HU*calibrated *= A* · *HU*measured, where *A* is a calibration coefficient determined for the specific combination of scanner model and kVp. The calibration factors can be derived from a large reference dataset using a phantom-less statistical approach - for instance, by comparing average vertebral attenuation in cohorts scanned at different kVp settings.

In an embodiment of the present invention, by calibrating to a common scale, the system ensures that BMD measurements are comparable across scans taken on different equipment and protocols. This approach obviates the need for inserting physical calibration phantoms in each scan, which is impractical for opportunistic screening.

The system and methods build on known techniques in the field of quantitative CT (QCT) where phantom-based or phantom-less corrections are used to standardize measurements.

### (E) Comparison to Normative Reference Data

In an embodiment of the present invention, the CT scan is of a mammal. In an embodiment of the present invention, the mammal is a human. In an embodiment of the present invention, the human is male. In an embodiment of the present invention, the human is female.

In an embodiment of the present invention, the calibrated trabecular attenuation (BMD surrogate) for each vertebra is then compared against reference curves or databases of normative values stratified by age and sex. Large population studies have established distributions of vertebral trabecular HU values by age group and sex.

For example, normative percentile curves for each vertebral level (e.g., T10, L1, etc.) can be used to determine where a patient's measurement lies in the population distribution. The software determines the patient's percentile (e.g., 20th percentile for age 70 female at L1) or compares against fixed threshold values that correspond to clinical definitions of osteoporosis. In some implementations, the system calculates the patient's values for multiple vertebrae (for instance, all vertebrae fully visible in the scan from T10 through L4). To provide a robust single summary value per patient, the median vertebra's attenuation can be selected - that is, the median of the percentiles across multiple vertebrae is used as the representative BMD measure for the patient. Using the median of multiple vertebral measurements mitigates the impact of any one abnormally high or low vertebra (for example, a fractured or degenerative vertebra).

In an embodiment of the present invention, the chosen value (median of a particular vertebra like L1 or the most median vertebra) is then used to determine the patient's BMD status. The system can output an age/sex percentile ranking and can flag patients below a certain percentile or threshold (for example, below the 12th percentile, which roughly corresponds to osteoporosis prevalence in older adults.

Hounsfield Units (HU) and T-scores are distinct non interchangeable measurement systems that relate to bone density. Hounsfield units are physical measures of tissue density i.e., a measure of X-ray attenuation in specific voxels on a CT scan. Hounsfield units are in a standardized, dimensionless scale, where pure water has an HU value of 0, air is defined as -1000 HU, bone and dense materials, have positive values (e.g., +700 to +3000 HU), while less dense materials like fat have negative values (e.g., -120 to -90 HU). CT artifacts, patient anatomy, and the specific CT scanner settings can influence HU values. In contrast, T-scores are statistical measures from a Dual-energy X-ray Absorptiometry (DXA) scan which can be used to classify bone health relative to a healthy population. T-scores are derived from total BMD measurements of a region in the DXA scan. The T-score represents the number of standard deviations a person's bone mineral density (BMD) is above or below the average BMD of a healthy, young adult of the same sex. DXA scans in patients with degenerative spine disease can result in elevated T-scores. A formula such as T-score = bHU + c is considered to be context-dependent and not typically useful in practice. However, a DXA scan is currently considered the gold standard for diagnosing osteoporosis.

In an embodiment of the present invention, the system and methods can also categorize the patient as having normal BMD, osteopenia, or osteoporosis by comparing to diagnostic cut-offs equivalent to those from DXA based criteria. A T-score from a DXA scan above -1.0 can indicate a patient has normal BMD. A T-score from a DXA scan between -1.0 to -2.5 can indicate mild to moderate bone loss and can result in a diagnosis of osteopenia. A T-score from a DXA scan below -2.5 can indicate low BMD and can result in a diagnosis of osteoporosis.

In summary, the invention combines advanced AI driven image segmentation, intelligent ROI selection, physics-based calibration, and epidemiologically-derived reference comparisons into a cohesive tool for opportunistic osteoporosis screening. It enables an automated, reproducible measurement of vertebral bone mineral density from existing CT scans, allowing identification of patients at risk for osteoporosis without additional dedicated scans. The method is more robust and accurate than prior approaches that used 2D single-slice measurements, because it analyzes a larger volume of bone and accounts for scanner variability and anatomical correctness of vertebral identification and automatically places the ROI. This system can be deployed in various forms, including as a software add-on to hospital PACS systems, a standalone application, or a cloud-based service, as described further below.

In an embodiment of the present invention, a method or system performs AI based BMD assessment from CT scans.

These and other aspects of the invention are evident in the drawings and in the description that follows.

### BRIEF DESCRIPTION OF THE DRAWINGS

This invention is described with respect to specific embodiments thereof. Additional features can be appreciated from the Figures in which:

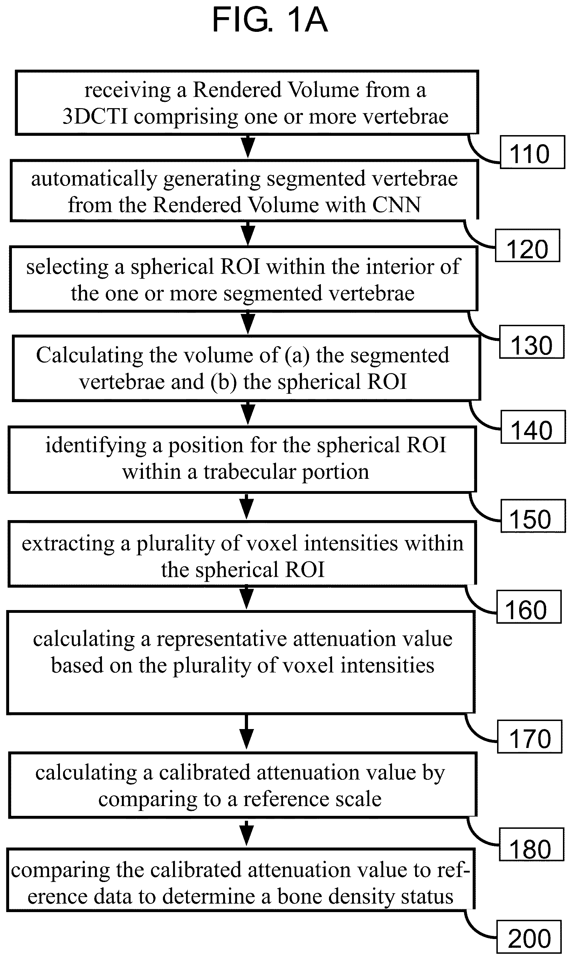

**FIG.1A** depicts a flow chart showing a schematic representation of a method to determine bone mineral density status of a segmented vertebrae, according to an embodiment of the invention;

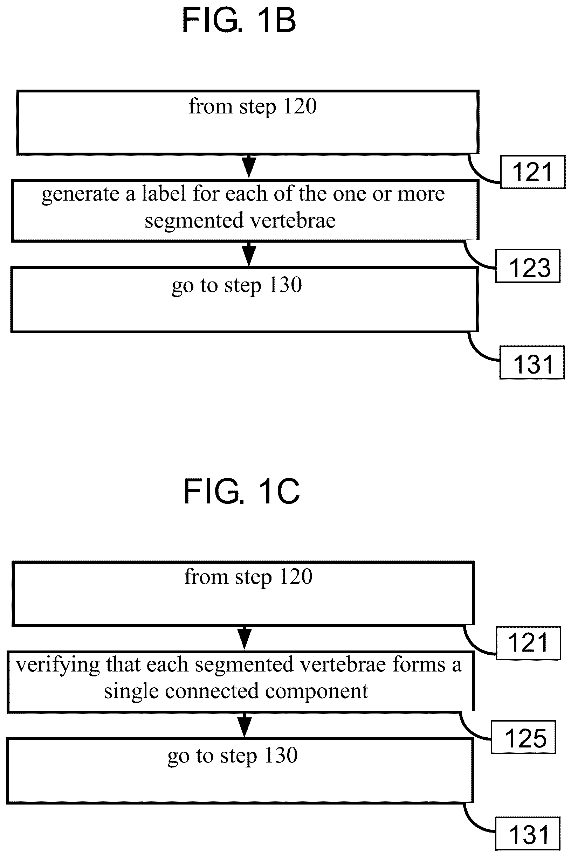

**FIG. 1B** depicts a flow chart showing a schematic representation of the method depicted in **FIG. 1A****,** where the segmented vertebrae are labelled, according to an embodiment of the invention;
**FIG. 1C** depicts a flow chart showing a schematic representation of the method depicted in **FIG. 1A****,** where each segmented vertebra is checked to determine that they form a single connected vertebra, according to an embodiment of the invention;

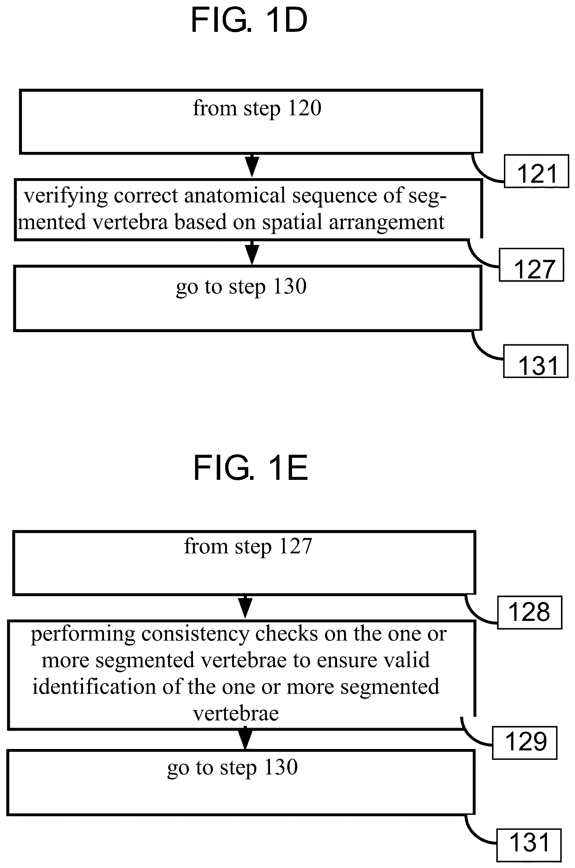

**FIG. 1D** depicts a flow chart showing a schematic representation of the method depicted in **FIG. 1A****,** where each segmented vertebra is checked to determine that they are in the correct anatomical sequence, according to an embodiment of the invention;
**FIG. 1E** depicts a flow chart showing a schematic representation of the method depicted in **FIG. 1D****,** where consistency checks are performed on the one or more segmented vertebrae to ensure valid identification of the one or more segmented vertebrae, according to an embodiment of the invention;

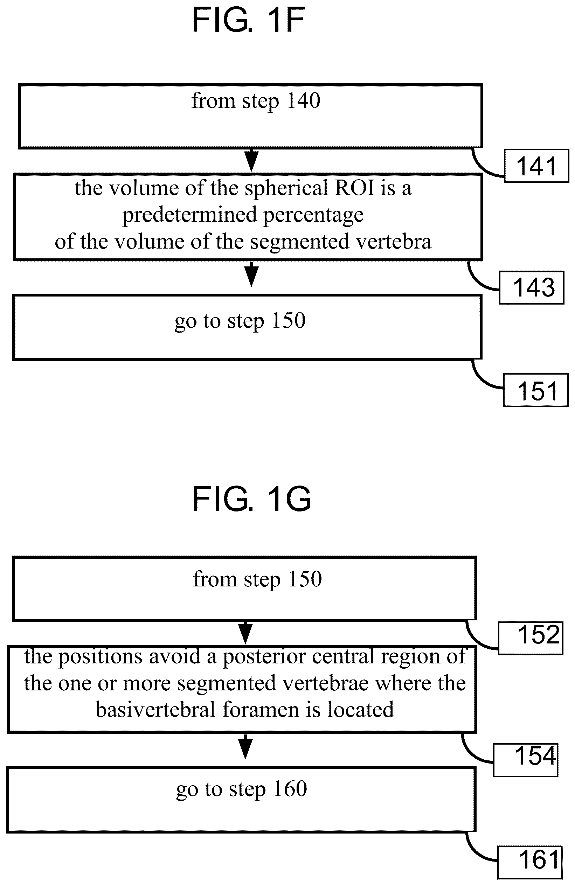

**FIG. 1F** depicts a flow chart showing a schematic representation of the method depicted in **FIG. 1A****,** where the volume of the three-dimensional spherical Region of Interest is a predetermined percentage of the volume of the one or more segmented vertebrae, according to an embodiment of the invention;
**FIG. 1G** depicts a flow chart showing a schematic representation of the method depicted in **FIG. 1A****,** where the position of the spherical ROI avoids the posterior central region of the one or more segmented vertebrae where a basivertebral foramen is located, according to an embodiment of the invention;

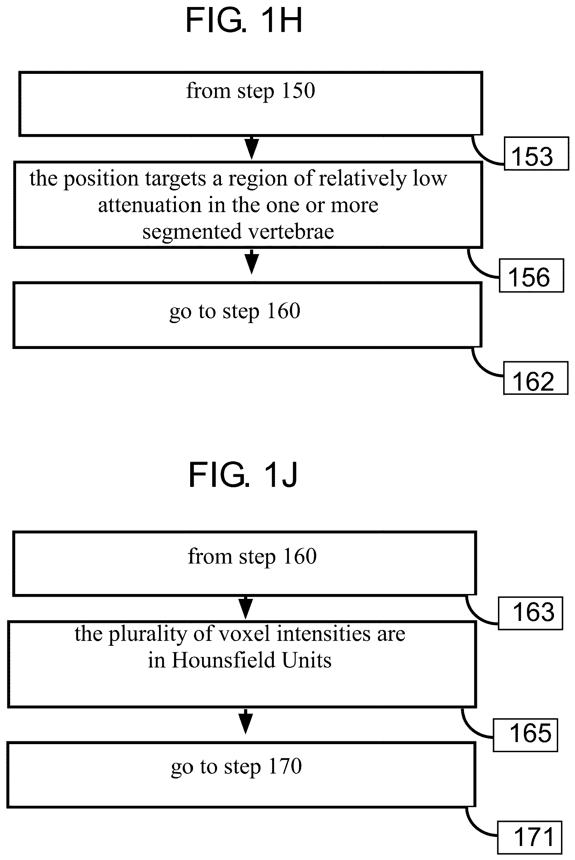

**FIG. 1H** depicts a flow chart showing a schematic representation of the method depicted in **FIG. 1A****,** where the position targets a region of relatively low attenuation within the one or more segmented vertebrae, according to an embodiment of the invention;
**FIG. 1J** depicts a flow chart showing a schematic representation of the method depicted in **FIG. 1A****,** where the plurality of voxel intensities is in Hounsfield Units, according to an embodiment of the invention;

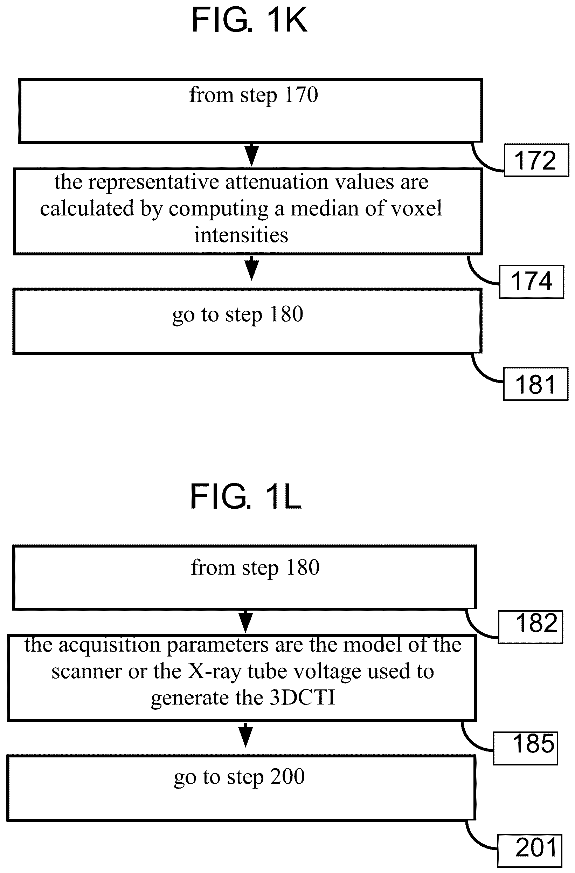

**FIG. 1K** depicts a flow chart showing a schematic representation of the method depicted in **FIG. 1A****,** where representative attenuation values are calculated by computing a median of the plurality of voxel intensities, according to an embodiment of the invention;
**FIG. 1L** depicts a flow chart showing a schematic representation of the method depicted in **FIG. 1A****,** where the acquisition parameters are the model of the scanner or the X-ray tube voltage used to generate the 3DCTI, according to an embodiment of the invention;

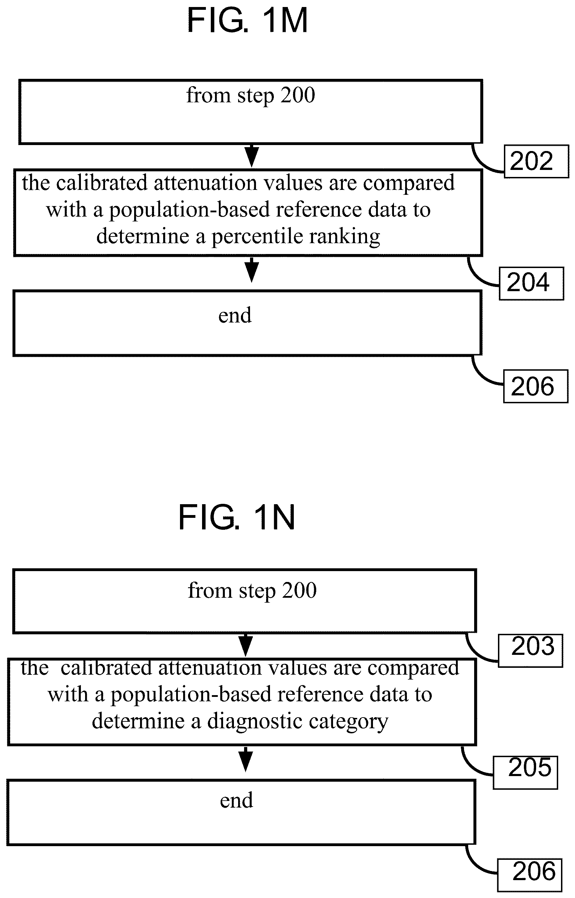

**FIG. 1M** depicts a flow chart showing a schematic representation of the method depicted in **FIG. 1A****,** where the calibrated attenuation values are compared with a population-based reference data stratified by one or both age and sex to determine a percentile ranking, according to an embodiment of the invention; and
**FIG. 1N** depicts a flow chart showing a schematic representation of the method depicted in **FIG. 1A****,** where the calibrated attenuation values are compared with a population-based reference data stratified by one or both age and sex to determine a diagnostic category, according to an embodiment of the invention.

### DEFINITIONS

The transitional term "comprising" is synonymous with "including", "containing", or "characterized by", is inclusive or open-ended and does not exclude additional, unrecited elements or method steps.

The transitional phrase "consisting of" excludes any element, step, or ingredient not specified in the claim, but does not exclude additional components or steps that are unrelated to the invention such as impurities ordinarily associated with a composition.

The transitional phrase "consisting essentially of' limits the scope of a claim to the specified materials or steps and those that do not materially affect the basic and novel characteristic(s) of the claimed invention.

The term "Study" refers in the usual and customary sense to the set of images produced by an examination. A Study consists of one or more images. The images can be grouped into one or more image series. Each image, each series, and the whole Study can have different parameters attached. For medical images these can be defined by the Digital Imaging and Communication in Medicine (DICOM) standard.

Some or all of the images in a Study can form one or more three-Dimensional "Volumes." For 3D CT modalities, often each individual image in the series corresponds to a volume, but that is not a requirement. For example, a cardiac CT may contain multiple 3D volumes covering the heart, each corresponding to a different point in the cardiac cycle, and all of the images belonging to all of these volumes being grouped into the same series.

The term "Hanging Protocol" refers in the usual and customary sense to specific conventions how X-Ray films are arranged at a light box.

The term "Display Protocol" refers in the usual and customary sense to the way images are displayed in a computer system, specifically the selection of the images to be displayed, the layout of the images, as well as the rendering parameters and styles.

The term "View" refers in the usual and customary sense to data corresponding to a digital image view of a Set of Images rendered with a given set of rendering parameters and rendering modes.

The term "Viewport" refers in the usual and customary sense to the logical part of the screen on the client computer in which a particular View is displayed, for example the user interface on the client computer can contain four rectangular Viewports of which three show a frontal, left, and bottom view respectively of a particular data, while the fourth viewer might show a 2D cross section through the same or a different data set.

The term "Sets of Images" or "Image Set" refers in the usual and customary sense to one or more images, selected based on the rules.

The term "Study Selection Rules" refers in the usual and customary sense to the rules used to select the studies to be displayed.

The term "Protocol Selection Rules" refers in the usual and customary sense to the rules used to select the layout of the images to be displayed.

The term "Image Set Rules" refers in the usual and customary sense to the rules used to form Image Sets **1165** from the images of one or more Study by applying selection, sorting, and breaking rules.

The term "Style Rules" refers in the usual and customary sense to the rules to determine which rendering type, rendering style, and rendering parameters are used for a particular Image Set in a particular viewer.

The terms "Volume Rendering" (verb) and "Rendered Volume" (RV) (noun) refers in the usual and customary sense to computer graphics visualization techniques that create e.g., a 2D and / or a 3D image from 3D image data sets, where a typical 3D image data set is a large number of 2D slice images acquired by a CT scanner and stored in a data structure. VR techniques that generate a RV include shaded volume, maximum intensity projection (MIP), oblique slicing or multi-planar reformats (MPR), axial/sagittal and coronal slice display, and thick slices (also called slabs).

The term "anatomical characteristic" includes the group consisting of one or more of spine, chest, abdomen, breast, shoulder, trapezius, arm, elbow, wrist, finger, pelvis, hip, fibula, knee, tibula, ankle, foot, neck, head, temporomandibular junction, face, brain, dentition, sinus, adrenals, retina, pituitary, and prostate. The anatomical characteristic can include the Body Part Examined. An anatomical characteristic can be either natural or pathologic. A natural anatomical characteristic of a patient would be the presence of seven cervical vertebrae. A pathologic anatomical characteristic of a patient would be the presence of only six cervical vertebrae.

The term "anatomical feature" refers to a medical condition, e.g., whether a fracture or bleeding is present in a given image or volume. An anatomical feature can be a fractured fibula, a herniated disc, urethral bleeding, e.g. bleeding with benign prostate hyperplasia, lacerated breast, Gun Shot Wound (GSW) to the chest, infection by *Treponema pertenue* giving rise to YAWS lesion in left distal leg. An anatomical feature is pathologic. In an embodiment of the invention, if ParameterA is fibula then the anatomical feature can be a fractured fibula.

The term "disease-based characteristic" can be selected from the type of disorder or disease being evaluated, e.g., a diagnosis of lung cancer. The disease-based characteristic can include the Body Part Examined. A disease-based characteristic can be pathologic.

The phrase "vertebral body" refers to the main, weight-bearing section of a single vertebra. The phrase "one or more vertebrae" refers to one vertebra or more vertebrae, meaning from one to the entire set of spinal bones in the vertebral column. The phrase "one or more segmented vertebrae" or "segmented vertebra" refers to each separate vertebra in the vertebral column.

The phrase "Convolutional Neural Networks" or CNN can be used to precisely identify vertebral bodies in three-Dimensional (3D) images.

A render server program is described in United States Patent No. 9,904,969, entitled "Multi-User Mult-GPU Render Server Apparatus and Methods", inventors M. Westerhoff et al., which issued February 27, 2018, and which is herein expressly incorporated by reference in its entirety and for all purposes. A rule based render server program is described in United States Patent No. 8,976,190, entitled "Method and System for Rule Based Display of Sets of Images", inventors M. Westerhoff et al., which issued March 10, 2015, and which is herein expressly incorporated by reference in its entirety and for all purposes. A program for visualizing DBT is described in United States Patent No. 9,984,478, entitled "Apparatus and Method for Visualizing Digital Breast Tomosynthesis and Other Volumetric Images", inventors M. Westerhoff et al., which issued May 29, 2018, and which is herein expressly incorporated by reference in its entirety and for all purposes. A program using Convolutional Neural Network analysis of image content based parameters is described in United States Patent No. 10,909,679, entitled "Method and System For Rule-Based Display of Sets of Images Using Image Content Derived Parameters", inventors M. Westerhoff et al., which issued October 1, 2020, and which is herein expressly incorporated by reference in its entirety and for all purposes. A program for transfer of large data sets is described in United States Patent No. 9,509,802, entitled "Method and System for Transferring Data to Improve Responsiveness when Sending Large Data Sets", inventors D Stalling et al., which issued November 29, 2016, and which is herein expressly incorporated by reference in its entirety and for all purposes. A program for anonymized data transfer is described in United States Patent No. 11,599,672, entitled "Method and System for Anonymized Display and Data Export", inventors D Stalling et al., which issued March 7, 2023, and which is herein expressly incorporated by reference in its entirety and for all purposes.

The phrase "basivertebral foramen" (BF) means a bone opening in the back wall of the vertebrae that contains the basivertebral veins and nerves. The BF is present in every vertebra of the spine.

The word "osteopenia" is a condition characterized by mild to moderate bone loss resulting in a lowered BMD. Osteopenia makes bones weaker and more susceptible to fractures. A T-score from a DXA scan above -1.0 can indicate a patient has a normal BMD. A T-score from a DXA scan between -1.0 to -2.5 can indicate mild to moderate bone loss and can result in a diagnosis of osteopenia.

The word "osteoporosis" is a condition characterized by severe bone loss resulting in a low BMD. Osteopenia makes bones weak, porous, and brittle and susceptible to higher risk of fractures from activities such as minor falls, bending, or even coughing. Fractures to the hip, spine, and wrist are common. A T-score from a DXA scan below -2.5 can indicate low BMD and can result in a diagnosis of osteoporosis.

The phrase "osteopenic trabecular region" means a part of bone that is affected by osteopenia. For example, the osteopenic travecular region is that part of the bone that thins due to osteopenia.

The phrase "vertebral trabecular bone region" means a part of bone that is inspected to determine if the patient is affected by osteopenia.

The phrase "spherical Regions of Interest" or "spherical ROI" means a 3D spherical volume for measuring bone tissue density within a specific area to extract quantitative data. By using a volumetric ROI (covering many voxels in 3D) instead of e.g., a single 2D slice patch, the influence of local inhomogeneities can be reduced and a more reliable measure of overall trabecular density can be obtained. The spherical ROI's center is chosen such that: (i) the sphere lies completely inside the vertebral body segmentation, (ii) the median HU value within the sphere is the lowest obtainable while satisfying that the sphere lies completely inside the vertebral body segmentation, and (iii) the sphere is located anterior to the basivertebral foramen (thus excluding the posterior vascular channel region).

The terms 'historical data', 'historical data set', refer in the usual and customary sense to data that has been previously measured. Historical data is existing X-ray or CT images (or other data) and does not comprise the step of creating new images of a patient. In an embodiment of the invention, the historical data set is a 3D CTI data set. In an alternative embodiment of the invention, the historical data set is a 3D RV generated from a 3D CTI data set. In an embodiment of the invention, the historical data set is a plurality of entries of 3D image data.

The "median HU value" is found by ranking the HU values and picking the middle one, if there is an even number of voxels, or an average of the two central values if there is an odd number of voxels.

The "representative attenuation value for a vertebra" can be computed once the spherical ROI is determined as the average HU value of all voxels inside the spherical ROI. In an embodiment of the invention, the average HU value is the median HU value of all voxels inside the spherical ROI. The "representative attenuation value for the one or more spherical ROI" is the average HU value of all voxels inside the spherical ROI. The average HU value for each vertebra can be calibrated to a standardized scale to generate the 'calibrated attenuation values'.

The "population-based reference data" refers in the usual and customary sense to a representative sample of a population with health norms that is used to assess and inform policy on osteopenia and osteoporosis.

The phrase "bone mineral density" (BMD) refers in the usual and customary sense to a measure of the calcium and minerals in bones, indicating their fracture risk, with lower density BMD making bones brittle.

The phrase "basivertebral veins" means the large, tortuous veins of the trabecular bone of vertebral bodies that drain into the internal and external vertebral venous plexuses. They emerge from the vertebral bodies horizontally through foramina in the bone. Anteriorly, they drain into the external vertebral venous plexuses and posteriorly, they converge into the basivertebral canal and drain into the anterior internal vertebral venous plexus.

The phrase "basivertebral venous plexus" refers in the usual and customary sense to a network of interconnected longitudinal channels located beneath the bony structures within the basivertebral canal. The plexus lacks a valve and relies on posture and respiration for drainage.

The hip joint is a ball-and-socket spheroidal synovial joint. The ball is the femoral head, and the socket is the acetabulum. The hip joint is the articulation of the pelvis with the femur, which connects the patient's axial skeleton with the lower extremity.

The phrase "femoral neck" refers in the usual and customary sense to the part of the thigh bone that connects the femoral head to the femoral shaft. The femoral neck is a flattened, pyramidal process of bone which is roughly cylindrical and flares out as it joins the shaft, it is located just below the ball of the hip joint.

The shoulder joint is a ball-and-socket spheroidal synovial joint. The shoulder joint is made up of the shoulder blade (scapula), the collar bone (clavicle) and upper arm (humerus). The acromioclavicular joint joins the top of your shoulder blade (acromion) and your collarbone together. The glenohumeral joint joins the rounded top of your upper arm bone into your shoulder blade (glenoid cavity of the scapula). The shoulder joint is formed by the articulation between the head of the humerus and the glenoid cavity of the scapula. It is the most mobile joint in the body.

The phrase "humeral neck" refers in the usual and customary sense to constricted part of the humerus bone that connects the head of the humerus to the body of the bone. It is located below the head of the humerus.

The knee joint refers in the usual and customary sense to a ball-and-socket spheroidal synovial joint. The knee joint is made up of the thigh bones (femur and fibula), shin bone (tibia), and the knee cap (patella). The tibiofemoral joint is a modified hinge synovial joint between the distal femur and the proximal tibia. The tibiofemoral joint is the weight bearing joint of the knee. The patella lies in an indentation of the femur known as the intercondylar groove. The patellofemoral joint is between the patella and the femur.

The phrase "distal femur" refers in the usual and customary sense to the lower part of the thigh bone, just above the knee joint. It's shaped like a trapezoid and flares out like an upside-down funnel.

The axial skeleton is the central core of the human skeleton, consisting of the bones that form the vertical axis of the body. The axial skeleton includes bones in your head, neck, back, pelvis, and chest. In contrast, the femur, the scapula, the clavicle and the humerus are all part of the appendicular skeleton.

The term "anatomical region" refers in the usual and customary sense to an area of a body. That is the anatomical region can include the head, the spine, the hip, the shoulder, and the knee. When the anatomical region is the spine, then the anatomical bones in the anatomical region are the one or more vertebrae. When the anatomical region is the hip, then the anatomical bones in the anatomical region are the one or more bones in the one or both hip joints. When the anatomical region is the shoulder, then the anatomical bones in the anatomical region are the one or more bones in the one or both shoulder joints. When the anatomical region is the knee, then the anatomical bones in the anatomical region are the one or more bones in the one or both knee joints.

The term "SegV" refers to a vertebral body (individual thoracic vertebrae bone and/or individual lumbar vertebrae bone) in a 3D region of a 3DCTI.

### INTRODUCTION

Osteoporosis is traditionally diagnosed by measuring BMD via DXA scans, but millions of CT scans taken for other reasons contain bone data that can be used to screen for osteoporosis opportunistically.

Prior research has demonstrated a correlation between CT attenuation of vertebrae and bone mineral density, offering thresholds to identify osteoporosis. For example, routine abdominal CT scans can be used for osteoporosis screening, proposing around 100 HU at L1 as a threshold indicative of osteoporosis risk. Later studies analyzed over 20,000 CT scans and reported normative trabecular attenuation values at L1, suggesting an approximate 90 HU threshold for osteoporosis at that level. In general, patients with low vertebral HU on CT are more likely to have low BMD on DXA.

However, early approaches often relied on manual or semi-automated techniques. The standard practice was to place a single 2D circular or elliptical ROI in the mid-section of one vertebral body (such as L1) on an axial CT slice and read the average HU value. This manual ROI placement is subject to variability. Results can differ depending on the slice level or exact ROI position, because trabecular bone mineral density is not uniform throughout a vertebra.

Small areas of sclerosis can skew the measurement. Indeed, it has been noted that there is significant variation in trabecular attenuation within a single vertebra when measured with a 2D ROI on one slice. Studies using a larger ROI volume can improve reproducibility. Furthermore, differences in CT scanner settings (tube voltage) and models can cause systematic differences in HU measurements of bone.

Previous commentators warned that attenuation in L1 trabecular bone can vary at different tube voltages, cautioning against using unadjusted HU values for osteoporosis screening across scans with dissimilar protocols. In other words, a patient's L1 HU measured at 80 kVp may not have the same attenuation as the same patient's L1 HU at 120 kVp, potentially leading to misclassification of osteoporosis if not corrected. It was further shown that automated opportunistic CT measures can predict future hip fractures.

One known challenge in these automated approaches was the calibration of HU values: early implementations sometimes applied a single conversion factor for all scans, which can introduce error if applied universally across different scanner models.

### DETAILED DESCRIPTION OF THE INVENTION

The present invention builds upon this prior work, addressing resolution of their limitations by introducing more refined calibration and robust ROI selection strategies.

Unexpectedly it was found the integration of advanced computational techniques enhance the accuracy of BMD estimation. It was also unexpectedly found the integration of advanced computational techniques enhance the robustness of BMD estimation. Further, it was unexpectedly found the integration of advanced computational techniques enhances the standardization of BMD estimation. In an embodiment of the present invention, the integration of advanced computational techniques enhance the accuracy of BMD estimation. In an alternative embodiment of the present invention, the integration of advanced computational techniques enhance the robustness of BMD estimation. In another embodiment of the present invention, the integration of advanced computational techniques enhance the standardization of BMD estimation

A deep learning-based CNN segmentation is used to precisely identify vertebral bodies in 3D, followed by anatomical consistency checks to detect and correct labeling errors, ensuring robustness against segmentation inaccuracies present in prior methods.

In an embodiment of the present invention, a 3D spherical ROI, sized as a fixed fraction of the vertebral volume, is strategically positioned using convolution-based optimization to locate the lowest-density region within the trabecular bone while avoiding anatomical artifacts such as the BF. Unlike prior approaches that positioned ROIs at the vertebral center or relied on single-slice measurements, which risk capturing denser cortical bone or structural irregularities, this method ensures a more precise assessment of trabecular attenuation.

In an embodiment of the present invention, the median HU value within the spherical ROI is used instead of the mean, improving measurement robustness by minimizing the influence of outliers such as focal sclerosis, calcifications, or artifacts that can skew the results in conventional averaging-based approaches.

In an embodiment of the present invention, the system selects the most representative vertebra for analysis based on percentile rankings across multiple vertebrae in a given patient. Instead of relying on a single predefined vertebra (e.g., L1) as in prior methods, this approach ensures that the measurement reflects overall trabecular bone health rather than being biased by localized pathology, fractures, or anomalies.

In an embodiment of the present invention, a two-step calibration process adjusts attenuation values for differences in scanner models and X-ray tube voltages (kVp), ensuring consistency across imaging protocols. This phantom-less standardization allows direct comparison of BMD estimates across different scans, eliminating variability introduced by hardware differences and imaging settings.

### System Architecture and Workflow

In an embodiment of the present invention, a software pipeline can process a 3D CT scan (axial image series) and output a BMD report. The pipeline may be integrated into a Picture Archiving and Communication System (PACS) or run on a separate workstation or cloud server. The steps of the method are as follows:

### 1. Input Acquisition

In an embodiment of the present invention, the system receives a CT scan volume that includes portions of the spine. This can be any CT of the chest, abdomen, or spine where at least one vertebral body is fully in view. The DICOM images are loaded into the processing software.

### 2. Vertebrae Segmentation using CNN

In an embodiment of the present invention, the entire 3D volume is passed through a trained convolutional neural network which outputs a segmentation mask identifying voxels that belong to bone (and specifically labels the vertebral bodies). The CNN is trained on a large set of manually labeled CTs to recognize vertebral bodies and differentiate them from other structures. It assigns an identifier to each vertebra (for example T10, T11, ..., L4, etc.) based on learned anatomical patterns (the network can output separate labels for each vertebral level). In some implementations, a two-stage approach may be used: first identify all vertebrae as a class, then apply a labeling scheme to distinguish levels. The result of this step is a set of 3D masks, one for each vertebral body present in the scan, along with an initial label (name/number of the vertebra).

### 3. Segmentation Consistency Checks

In an embodiment of the present invention, the system then evaluates the segmentation for quality:

### 3A. Connectivity Check

In an embodiment of the present invention, for each vertebral mask, the software checks that the mask is one connected 3D region. If a vertebra's mask is fragmented into multiple pieces (which can happen if the CNN mistakenly split one vertebra or if part of the vertebra is missing at scan edges), the system can flag this as an error. In edge cases where a vertebra is at the superior or inferior extreme of the scan range (cut off by the scan boundary), multiple components might appear (e.g., only the top half of a vertebra is present). Those boundary vertebrae can be excluded from analysis, since they are not fully captured. Ensuring single-component segmentation for each vertebra confirms that the CNN correctly identified whole vertebral bodies.

### 3B. Sequential Order and Position Check

In an embodiment of the present invention, the relative positions of the labeled vertebrae are verified to ensure proper ordering (e.g., T12 should be below T11 and above L1, aligned roughly along the spinal axis). The software computes each vertebra's center of mass (or centroid of the mask) in 3D coordinates. Then it checks that as one moves down the spine, the centroids progress consistently without large jumps or reversals. The system may calculate the angle of the line connecting successive centroids and the distance between them to verify a smooth anatomical curve: The angle should be greater than 90 degrees and the distance from one set of vertebrae to the next should not differ by more than a pre-defined factor, e.g. 1.5x.

In an embodiment of the present invention, if two adjacent identified "vertebrae" have centroids that are too far apart or not aligned vertically, it can indicate a missed vertebra or a labeling mistake (e.g., skipping a vertebra number). In an embodiment of the present invention, if the order or count is inconsistent, the software can relabel using expected spacing. In an alternative embodiment of the present invention, if the order or count is inconsistent, the software can mark the case as requiring manual review. This geometric sanity check ensures that each vertebral body is correctly identified by level.

In an embodiment of the present invention, if the segmentation passes these consistency checks, the process continues; otherwise, the system may either correct minor issues (like merge small fragmented components, or adjust a label) or abort processing for that scan and output an error indicating the automated analysis was not confident.

### 4. Vertebral Volume Calculation

In an embodiment of the present invention, for each vertebra that passed validation, the volume (in cubic millimeters or cubic centimeters) of the trabecular region is computed from the segmentation mask. The volume is simply the count of voxels in the mask times the volume per voxel. This volumetric information is used to size the ROI in the next step. In an embodiment of the present invention, the volumetric information can also serve as an indicator (e.g., a very small volume might indicate an incomplete or damaged vertebra).

### 5. Spherical ROI Placement

In an embodiment of the present invention, the system determines a spherical region of interest inside each vertebra for measuring attenuation.

### 5A.

In an embodiment of the present invention, the target ROI volume is set as a percentage of the vertebra's volume (e.g., approximately 5%, where in this range approximately means plus or minus 30%, i.e., 3.5% to 6.5%). In an embodiment of the present invention, the proportion can be adjusted. In an embodiment of the present invention, 5% has been found effective in capturing a representative sample while being small enough to fit entirely within most vertebrae. In an embodiment of the present invention, the predetermined percentage is between a lower limit of approximately three per cent, and an upper limit of approximately seven per cent. In an alternative embodiment of the present invention, the predetermined percentage is between a lower limit of approximately two per cent, and an upper limit of approximately eight per cent.

### 5B.

In an embodiment of the present invention, an algorithm searches within the vertebral mask to find the optimal location for this sphere. The criteria include:

### 5B(i).

In an embodiment of the present invention, the sphere can fit entirely within the vertebra mask. In an alternative embodiment of the present invention, the sphere fits entirely within the vertebra mask so that none of its voxels fall outside the segmented bone.

### 5B(ii).

In an embodiment of the present invention, the sphere's average density (HU) is as low as possible. In an alternative embodiment of the present invention, an algorithm can scan the interior of the vertebra, evaluating candidate sphere positions to find a location that minimizes mean HU. In another alternative embodiment of the present invention, an algorithm can scan the interior of the vertebra, use an optimization strategy to find a location that minimizes mean HU. In various embodiments of the present invention, this tends to place the ROI in the most osteopenic (least dense) trabecular region of the vertebra.

### 5B(iii).

In an embodiment of the present invention, the sphere can be positioned anteriorly relative to the center of the vertebra. In an embodiment of the present invention, the sphere can be positioned avoiding the posterior third of the vertebral body. In an embodiment of the present invention, the sphere can be positioned where the BF and surrounding venous plexus are located. Positioning the sphere in the above manner is important because the basivertebral venous plexus can appear as a localized low-density or high-density area depending on blood or sclerosis around it, which can otherwise confound the HU measurement.

### 5C.

In an embodiment of the present invention, once the sphere position is chosen, the ROI sphere is defined, and its voxels are recorded.

### 6. Attenuation Measurement.

In an embodiment of the present invention, with the ROI in place, the system extracts all the HU values of voxels inside the spherical ROI. In an embodiment of the present invention, with the ROI in place, the system then calculates the median HU value of these voxels. In an embodiment of the present invention, the median is found by ranking the values and picking the middle one. In an embodiment of the present invention, if there is an even number of voxels, the median is an average of the two central values. The choice of median provides robustness to any outlier values, such as a small patch of sclerosis that might raise the mean but can only occupy a small fraction of the ROI.

In an embodiment of the present invention, the median HU is taken as the representative trabecular attenuation for that vertebra.

### 7. Calibration of HU to BMD Scale.

In an embodiment of the present invention, the raw median HU for each vertebra is then calibrated to a standardized scale. In one embodiment, two calibration factors are applied multiplicatively: one for the scan's kVp, and one for the scanner model. For example, if the scan was done at 100 kVp on Scanner Model X, the system will apply:

**7A.**

In an embodiment of the present invention, the HU_adjusted_for_kVp = HU_median * CkVp, where CkVp=100-120</sub> is a coefficient converting 100 kVp to the 120 kVp reference. In an embodiment of the present invention, if bone appears denser at lower kVp, this coefficient can be <1.0 to scale down, or >1.0 to scale up.

### 7B.

In an embodiment of the present invention, then the HU_adjusted_for_scanner equals HU_adjusted_for_kVp * CModelX , where CModelX is a factor for that specific scanner relative to the reference scanner model. These factors can be determined ahead of time by analyzing large datasets as described earlier. Tables of calibration coefficients for common kVp settings and scanner manufacturers can be stored in the system. The result of this step is a calibrated HU value for each vertebra that represents what the attenuation can be if the scan had been performed under reference conditions. By performing this calibration, the method aligns the measurements with a consistent BMD scale, addressing the known issue that different CT settings yield different attenuation readings.

### 7C.

In an embodiment of the present invention, a more complex function or even a machine learning model can be used for calibration (taking into account one or more of kVp, reconstruction kernel, and patient size).

### 8. Analysis of Multiple Vertebrae (if available):

The system will repeat steps 5-7 (see paragraphs [0104] to [0128] above) for each vertebral body that is fully within the scan range (e.g., T12, L1, L2, L3, etc., depending on the scan). This yields a set of calibrated attenuation values for the patient's spine. If more than one vertebra is measured, the system can aggregate these to improve reliability. One preferred approach is to identify the most median vertebrae and use it as the patient's representative value.

For example, if T12, L1, and L2 are measured, sort those three values and take the middle value. This approach helps avoid cases where a single abnormally low value (perhaps from a localized bone issue or imaging artifact) can falsely classify the patient as osteoporotic, or a single abnormally high value (perhaps due to degenerative sclerosis in one vertebra) can mask osteoporosis in the others. The system can be configured to use a particular vertebra or a combination. In an example embodiment, the software selects the L1 vertebra if it is present and fully captured; if not, it uses the next available (T12 or L2), or uses an average of the nearest two, etc. The median-of-multiple approach is generally preferred for robustness if multiple levels are available.

### 9. Comparison to Reference Data.

The final calibrated attenuation value (or values for each vertebra) are compared to reference standards. The invention includes a database of normative attenuation values by vertebral level, age, and sex (derived from population studies or clinical data).

For each vertebra measured, the system can determine the patient's percentile. It can also determine an overall percentile or category for the patient. For instance, the system might output:

### 9A.

L1 trabecular attenuation = 55 HU, which is the 8th percentile for a 65-year-old female (significantly below average).

### 9B.

By established criteria, this is consistent with osteoporosis since 58 HU at L1 is below the cut-off for osteoporosis of 68HU as per reference data.

### 10. Output Generation.

The system generates a report or data file with the results. This can include the numerical values (raw and calibrated HU), the reference percentile, and a suggested diagnostic category. It may also include a visual image with the ROI marked on a representative CT slice for review, or a 3D rendering of the spine with color-coding of BMD. In a PACS integration, the results can be sent as secondary capture images or DICOM Structured Reports attached to the patient's study. In a cloud service, the results might be delivered via a web interface or an API to an electronic health record.

Throughout this process, the method is fully automatic. It does not require a technician to place ROIs or calibrate the machine, making it suitable for high-throughput screening of large numbers of CT scans. The results are presented to the radiologist or other physician for confirmation before making a final diagnosis or treatment decision.

### Embodiments.

The invention can be embodied in several forms to fit into clinical workflows:

### Standalone Processing Service

In an embodiment of the present invention, the software operates on a dedicated workstation or server within a radiology department. CT scans from the scanner or PACS are forwarded to this system (for example, via DICOM network transfer). The system processes each scan to compute the BMD metrics and then returns a report. Radiology staff can then review the results and incorporate them into patient evaluations. This standalone system can be a software package installed on existing hardware or sold as an appliance (hardware + software).

### PACS Integration.

In an embodiment of the present invention, the system can be integrated directly into a hospital's PACS or advanced visualization workstation. For instance, when a radiologist opens a spine or body CT in the PACS, they can trigger the "BMD Analysis" which invokes the algorithm on that series. The integration can also be automatic: as soon as a new CT scan is saved to PACS, the system detects if spine vertebrae are present and, if so, performs the analysis in the background. Results can then be attached to the study for the radiologist to see. Integration ensures the tool fits seamlessly into existing image interpretation workflows.

### Cloud-Based Deployment

In an embodiment of the present invention, the analysis is carried out on cloud servers. CT scans (de-identified or via secure VPN) are uploaded to a cloud service that runs the AI algorithm. This is useful for institutions that prefer not to maintain on-premises hardware or for providing the service to many sites. A cloud deployment also facilitates continuous learning: the system can aggregate data from all processed scans to further refine normative databases or improve the CNN through federated learning (if permitted).

Each embodiment leverages the same core algorithm but offers different advantages. The standalone and PACS-integrated versions allow for real-time use within a hospital with minimal data leaving the facility. The cloud version offers scalability and easier updates (since improving the AI model or calibration curves can be done centrally). Importantly, regulatory compliance and data privacy are maintained in each case.

### Examples.

### Opportunistic Osteoporosis Screening

The primary use case is analyzing existing CT scans (taken for any indication, e.g. trauma, pain, cancer staging, etc.) to identify patients with low bone mineral density who might otherwise be unaware of their condition. For example, an older patient gets an abdominal CT for kidney stones; the radiologist runs this tool and discovers the patient's spine shows low BMD, prompting a recommendation for a dedicated bone health evaluation. This opportunistic screening can significantly increase osteoporosis detection rates without additional radiation or scan appointments.

### Fracture Risk Assessment

The system's results can be used alongside clinical risk factors to assess fracture risk. Doctors might use the vertebral attenuation measurement in calculators or scoring systems. A very low HU value in the spine can suggest high fracture risk, leading to interventions (like starting osteoporosis medication or fall-prevention strategies). Research has shown that CT-based bone measures are predictive of future fractures, so this tool can aid in risk stratification.

### Baseline and Monitoring

Patients who undergo serial CT scans (for example, cancer patients who get periodic CTs) can have their bone mineral density monitored over time using this invention. The calibrated values allow comparison across scans even if different machines or protocols were used. This can effectively provide a "CT bone densitometry" track record without dedicated scans. Clinicians can observe if a patient's bone mineral density is dropping significantly and take action.

### Population Health and Research

On a larger scale, hospitals or health systems can apply this tool to their archive of CT images to gather epidemiological data on bone health. Because the method is automated and scalable, it can scan through thousands of prior CTs to establish normative BMD ranges for various populations.

Public health researchers can use such data to study osteoporosis prevalence in different demographic groups or the effect of certain treatments on bone mineral density incidentally.

### ADVANTAGEOUS ASPECTS OF THE INVENTION

The described system has several important advantages over prior techniques:

### Automation and Efficiency

In an embodiment of the present invention, the system eliminates the need for manual ROI placement, reducing labor and inter-observer variability. Once deployed, it can process scans rapidly and consistently, ensuring that no potential osteoporosis case is missed due to human oversight. This is crucial given the large number of CT scans performed; even a modest fraction being analyzed can yield many detections of previously unknown osteoporosis.

### Improved Accuracy and Reproducibility

By using a 3D ROI and median value, the measurement is more reproducible across different slices or different scans of the same patient.

In an embodiment of the present invention, the approach is less sensitive to small local anomalies in the bone. The consistency checks on segmentation also ensure the anatomy is correctly identified, preventing gross errors.

### Sensitivity to Low BMD

In one embodiment of the present invention, the strategy of targeting the lowest-density region in the trabecular bone (while avoiding artifacts) makes the measurement more sensitive to osteoporosis. Traditional single-slice methods might miss such low-density spots or dilute them with higher-density bone, yielding false-negatives. Our method effectively seeks the "weakest point" of the bone's internal structure to gauge overall bone health, which correlates with fracture susceptibility.

### Calibration Across Scanners

A major advantage is the built-in calibration that allows data from any CT scanner or protocol to be interpreted on the same scale. This means the tool can be deployed across different hospitals with varying equipment and still produce comparable results. It also means that as a patient goes from one facility to another, their CT-based BMD measurement remains meaningful. Prior art either ignored these differences or required phantom scans; our approach makes it seamless and based on statistical knowledge gleaned from real-world data.

### Integration and Workflow

The invention is designed to integrate with existing medical imaging workflows. Radiologists and clinicians can get this additional information without significant extra effort. The outputs can be presented in familiar formats (e.g., as part of the radiology report or as annotated images). This encourages adoption by clinicians, which is essential for opportunistic screening to actually translate into improved patient care.

Moreover, because it uses existing scans, it adds no risk to the patient (no extra radiation) and no inconvenience, which improves patient acceptance.

### Versatility and Extensibility

While the current focus is on vertebral bodies for osteoporosis, the same framework can be extended to other bones (e.g., femoral neck on hip CTs) or other clinical metrics (like detecting high density in bone metastases or quality of bone grafts). The modular design (segmentation → ROI → measure → calibrate → compare to reference) can be adapted, demonstrating the flexibility of the approach.

**FIG. 1A** depicts a flow chart showing a schematic representation of a method to determine bone mineral density status of a segmented vertebrae. In step **110** a three-Dimensional Computer Tomography Image (3DCTI) comprising one or more vertebrae is received. In step **120** one or more segmented vertebrae are automatically generated from the 3DCTI with a Convolutional Neural Network. In step **130** a three-Dimensional Spherical Region of Interest (3DSROI) is selected where the 3DSROI is within the interior of the one or more segmented vertebrae. In step **140** a volume of the three-dimensional Spherical Region of Interest (3DSROIV) is calculated. In step **150** a position for the 3DSROI is identified, where the 3DSROI lies entirely within a trabecular portion of the one or more segmented vertebrae. In step **160** a plurality of voxel intensities within the 3DSROI are extracted. In step **170** a representative attenuation value based on the plurality of voxel intensities is calculated. In step **180** a calibrated attenuation value is calculated based on comparing the representative attenuation value to a reference scale to compensate for differences in one or more acquisition parameters of the 3DCTI. In step **200** the calibrated attenuation value is compared to a population-based reference data to determine a bone mineral density status of the one or more segmented vertebrae.

**FIG. 1B** depicts a flow chart showing a schematic representation of the method depicted in **FIG. 1A****,** where in step 121 (after step 120), in step 123 the segmented vertebrae are labelled and then in step 131 the method returns to step 130 of the method depicted in **FIG. 1A****.** **FIG. 1C** depicts a flow chart showing a schematic representation of the method depicted in **FIG. 1A****,** where in step 121 (after step 120), in step 125 each segmented vertebra is checked to determine that they form a single connected vertebra and then in step 131 the method returns to step 130 of the method depicted in **FIG. 1A****.** **FIG. 1D** depicts a flow chart showing a schematic representation of the method depicted in **FIG. 1A****,** where in step 121 (after step 120), in step 127 each segmented vertebra is checked to determine that they are in the correct anatomical sequence and then in step 131 the method returns to step 130 of the method depicted in **FIG. 1A****.** **FIG. 1E** depicts a flow chart showing a schematic representation of the method depicted in **FIG. 1D****,** where in step 128 (after step 127), in step 120 consistency checks are performed on the one or more segmented vertebrae to ensure valid identification of the one or more segmented vertebrae and then in step 131 the method returns to step 130 of the method depicted in **FIG. 1A****.** **FIG. 1F** depicts a flow chart showing a schematic representation of the method depicted in **FIG. 1A****,** where in step 132 (after step 130), in step 133 the volume of the three-dimensional spherical Region of Interest is a predetermined percentage of a volume of the one or more segmented vertebrae and then in step 141 the method returns to step 140 of the method depicted in **FIG. 1A****.** **FIG. 1G** depicts a flow chart showing a schematic representation of the method depicted in **FIG. 1A****,** where the method is modified in step 152 (which follows step 150), and in step 154 where the position of the spherical ROI avoids the posterior central region of the one or more segmented vertebrae where a basivertebral foramen is located and then in step 161 the method returns to step 160 of the method depicted in **FIG. 1A****.** **FIG. 1H** depicts a flow chart showing a schematic representation of the method depicted in **FIG. 1A****,** where the method is modified in step 153 (which follows step 150), and in step 156 where the position targets a region of relatively low attenuation within the one or more segmented vertebrae and then in step 162 the method returns to step 160 of the method depicted in **FIG. 1A****.** **FIG. 1J** depicts a flow chart showing a schematic representation of the method depicted in **FIG. 1A****,** where the method is modified in step 163 (which follows step 160), and in step 165 where the plurality of voxel intensities is in Hounsfield Units and then in step 171 the method returns to step 170 of the method depicted in **FIG. 1A****.** **FIG. 1K** depicts a flow chart showing a schematic representation of the method depicted in **FIG. 1A****,** where the method is modified in step 172 (which follows step 170), and in step 174, where representative attenuation values are calculated by computing a median of the plurality of voxel intensities and then in step 181 the method returns to step 180 of the method depicted in **FIG. 1A****.** **FIG. 1L** depicts a flow chart showing a schematic representation of the method depicted in **FIG. 1A****,** where the method is modified in step 182 (which follows step 180), and in step 185, where the acquisition parameters are the model of the scanner or the X-ray tube voltage used to generate the 3DCTI, according to an embodiment of the invention and then in step 201 the method returns to step 200 of the method depicted in **FIG. 1A****.** **FIG. 1M** depicts a flow chart showing a schematic representation of the method depicted in **FIG. 1A****,** where the method is modified in step 202 (which follows step 200), and in step 204, where the calibrated attenuation values are compared with a population-based reference data stratified by one or both age and sex to determine a percentile ranking and then in step 206 the method ends. **FIG. 1N** depicts a flow chart showing a schematic representation of the method depicted in **FIG. 1A****,** where the method is modified in step 203 (which follows step 200), and in step 205 where the calibrated attenuation values are compared with a population-based reference data stratified by one or both age and sex to determine a diagnostic category and then in step 206 the method ends.

Overall, this invention provides a comprehensive solution for opportunistic bone health assessment using CT scans, addressing prior deficiencies and enabling widespread screening. By catching osteoporosis earlier, it can help initiate treatment sooner, ultimately aiming to reduce fracture incidence and improve patient outcomes.

Described above are methods and systems for implementing an AI based BMD assessment from CT scans. The foregoing description of embodiments of the methods, systems, and components of the present invention has been provided for the purposes of illustration and description. It is not intended to be exhaustive or to limit the invention to the precise forms disclosed. Many modifications and variations will be apparent to one of ordinary skill in the relevant arts. For example, steps performed in the embodiments of the invention disclosed can be performed in alternate orders, certain steps can be omitted, and additional steps can be added. The embodiments were chosen and described in order to best explain the principles of the invention and its practical application, thereby enabling others skilled in the art to understand the invention for various embodiments and with various modifications that are suited to the particular used contemplated. Other embodiments are possible and are covered by the invention. Such embodiments will be apparent to persons skilled in the relevant art(s) based on the teachings contained herein. The breadth and scope of the present invention should not be limited by any of the above-described exemplary embodiments, but should be defined only in accordance with the following claims and their equivalents.

### FURTHER EMBODIMENTS OF THE INVENTION

A computer implemented method for determining bone mineral density in trabecular bone of vertebral bodies using CT scan data, the method comprising: (a) acquiring a three-dimensional CT image volume that includes one or more vertebrae; (b) processing the CT image volume with a convolutional neural network (CNN) to automatically segment and label each vertebral body present in the volume; (c) performing consistency checks on the segmented vertebrae to ensure valid identification, including verifying that each segmented vertebral body forms a single connected component in the segmentation and that the labeled vertebrae follow a correct anatomical sequence based on their spatial arrangement; (d) for each correctly segmented vertebral body, calculating its volume and automatically selecting a three-dimensional spherical region of interest (ROI) within the interior of that vertebral body, the spherical ROI having a volume that is a predetermined percentage of the vertebra's volume; (e) positioning the spherical ROI such that it lies entirely within the trabecular portion of the vertebra and avoids the posterior central region of the vertebral body where a BF is located, the positioning further chosen to target a region of relatively lowest attenuation within the vertebra; (f) extracting the voxel intensities (Hounsfield Units) from the CT image within the spherical ROI and calculating a representative attenuation value for the vertebra by computing a median of said voxel intensities; (g) calibrating the representative attenuation value to a reference scale to compensate for differences in CT acquisition parameters, by applying one or more predetermined calibration factors corresponding to the CT scanner model and X-ray tube voltage used for the image volume; and (h) comparing the calibrated attenuation value for the vertebra to population-based reference data stratified by age and sex to determine a percentile ranking or diagnostic category indicating the bone mineral density status of the vertebra.

A system of Deploying in PACS or Cloud to process large numbers of studies in an automated fashion comprising: one or more processors and memory storing instructions that, when executed by the one or more processors, cause the system to: (a) acquire a three-dimensional CT image volume that includes one or more vertebrae; (b) process the CT image volume with a convolutional neural network (CNN) to automatically segment and label each vertebral body present in the volume; (c) perform consistency checks on the segmented vertebrae to ensure valid identification, including verifying that each segmented vertebral body forms a single connected component in the segmentation and that the labeled vertebrae follow a correct anatomical sequence based on their spatial arrangement; (d) for each correctly segmented vertebral body, calculate its volume and automatically selecting a three-dimensional spherical region of interest (ROI) within the interior of that vertebral body, the spherical ROI having a volume that is a predetermined percentage of the vertebra's volume; (e) position the spherical ROI such that it lies entirely within the trabecular portion of the vertebra and avoids the posterior central region of the vertebral body where a BF is located, the positioning further chosen to target a region of relatively lowest attenuation within the vertebra; (f) extract the voxel intensities (Hounsfield Units) from the CT image within the spherical ROI and calculating a representative attenuation value for the vertebra by computing a median of said voxel intensities; (g) calibrate the representative attenuation value to a reference scale to compensate for differences in CT acquisition parameters, by applying one or more predetermined calibration factors corresponding to the CT scanner model and X-ray tube voltage used for the image volume; and (h) compare the calibrated attenuation value for the vertebra to population-based reference data stratified by age and sex, where the system further generates a report indicating a patient's bone mineral density status based on the analysis of one or more vertebrae in an input CT scan.

A method of Vertebral Segmentation by a CNN comprising: (a) acquiring a three-dimensional CT image volume that includes one or more vertebrae; (b) processing the CT image volume with a convolutional neural network (CNN) to automatically segment and label each vertebral body present in the volume; (c) performing consistency checks on the segmented vertebrae to ensure valid identification, including verifying that each segmented vertebral body forms a single connected component in the segmentation and that the labeled vertebrae follow a correct anatomical sequence based on their spatial arrangement; (d) for each correctly segmented vertebral body, calculating its volume and automatically selecting a three-dimensional spherical region of interest (ROI) within the interior of that vertebral body, the spherical ROI having a volume that is a predetermined percentage of the vertebra's volume; (e) positioning the spherical ROI such that it lies entirely within the trabecular portion of the vertebra and avoids the posterior central region of the vertebral body where a BF is located, the positioning further chosen to target a region of relatively lowest attenuation within the vertebra; (f) extracting the voxel intensities (Hounsfield Units) from the CT image within the spherical ROI and calculating a representative attenuation value for the vertebra by computing a median of said voxel intensities; (g) calibrating the representative attenuation value to a reference scale to compensate for differences in CT acquisition parameters, by applying one or more predetermined calibration factors corresponding to the CT scanner model and X-ray tube voltage used for the image volume; (h) comparing the calibrated attenuation value for the vertebra to population-based reference data stratified by age and sex, where the CNN is trained to identify and label vertebral bodies across a range of spinal levels, and the CNN outputs a labeled segmentation distinguishing individual vertebrae (thoracic and/or lumbar); and (h) excluding from analysis any vertebrae that are only partially present at the edges of the CT field of view.

A method of using Segmentation Consistency Checks to verifying that the sequence of vertebrae is ordered and aligned consistent with normal spine anatomy comprising: (a) acquiring a three-dimensional CT image volume that includes one or more vertebrae; (b) processing the CT image volume with a convolutional neural network (CNN) to automatically segment and label each vertebral body present in the volume; (c) performing consistency checks on the segmented vertebrae to ensure valid identification, including verifying that each segmented vertebral body forms a single connected component in the segmentation and that the labeled vertebrae follow a correct anatomical sequence based on their spatial arrangement; (d) for each correctly segmented vertebral body, calculating its volume and automatically selecting a three-dimensional spherical region of interest (ROI) within the interior of that vertebral body, the spherical ROI having a volume that is a predetermined percentage of the vertebra's volume; (e) positioning the spherical ROI such that it lies entirely within the trabecular portion of the vertebra and avoids the posterior central region of the vertebral body where a BF is located, the positioning further chosen to target a region of relatively lowest attenuation within the vertebra; (f) extracting the voxel intensities (Hounsfield Units) from the CT image within the spherical ROI and calculating a representative attenuation value for the vertebra by computing a median of said voxel intensities; (g) calibrating the representative attenuation value to a reference scale to compensate for differences in CT acquisition parameters, by applying one or more predetermined calibration factors corresponding to the CT scanner model and X-ray tube voltage used for the image volume; (h) comparing the calibrated attenuation value for the vertebra to population-based reference data stratified by age and sex; and (i) performing consistency checks comprises: (j) determining connectedness of each vertebral body's segmented region in three dimensions and rejecting any segmentation where a single vertebral body is divided into multiple disjoint segments; and (k) calculating spatial coordinates for each vertebral body (including at least the centroids or centers of mass of each segmented vertebra) and verifying that the sequence of vertebrae is ordered and aligned consistent with normal spine anatomy, flagging cases of out-of-order labels, missing vertebrae, or irregular spacing/angles between adjacent vertebrae.

A method of using Adaptive Spherical ROI Placement to identify a location that minimizes the average Hounsfield Unit value within the sphere while remaining a fixed distance away from the cortical bone boundaries and anterior to the BF region, comprising: (a) acquiring a three-dimensional CT image volume that includes one or more vertebrae; (b) processing the CT image volume with a convolutional neural network (CNN) to automatically segment and label each vertebral body present in the volume; (c) performing consistency checks on the segmented vertebrae to ensure valid identification, including verifying that each segmented vertebral body forms a single connected component in the segmentation and that the labeled vertebrae follow a correct anatomical sequence based on their spatial arrangement; (d) for each correctly segmented vertebral body, calculating its volume and automatically selecting a three-dimensional spherical region of interest (ROI) within the interior of that vertebral body, the spherical ROI having a volume that is a predetermined percentage of the vertebra's volume; (e) positioning the spherical ROI such that it lies entirely within the trabecular portion of the vertebra and avoids the posterior central region of the vertebral body where a BF is located, the positioning further chosen to target a region of relatively lowest attenuation within the vertebra; (f) extracting the voxel intensities (Hounsfield Units) from the CT image within the spherical ROI and calculating a representative attenuation value for the vertebra by computing a median of said voxel intensities; (g) calibrating the representative attenuation value to a reference scale to compensate for differences in CT acquisition parameters, by applying one or more predetermined calibration factors corresponding to the CT scanner model and X-ray tube voltage used for the image volume; (h) comparing the calibrated attenuation value for the vertebra to population-based reference data stratified by age and sex; and (i) identifying a predetermined percentage of the vertebra's volume for the spherical ROI is between 1% and 10%, and where selecting and positioning the spherical ROI further comprises identifying a location that minimizes the average Hounsfield Unit value within the sphere while remaining a fixed distance away from the cortical bone boundaries and anterior to the BF region.

A method of using a Median Attenuation Value to yield a lower and more reliable trabecular attenuation measure, comprising: (a) acquiring a three-dimensional CT image volume that includes one or more vertebrae; (b) processing the CT image volume with a convolutional neural network (CNN) to automatically segment and label each vertebral body present in the volume; (c) performing consistency checks on the segmented vertebrae to ensure valid identification, including verifying that each segmented vertebral body forms a single connected component in the segmentation and that the labeled vertebrae follow a correct anatomical sequence based on their spatial arrangement; (d) for each correctly segmented vertebral body, calculating its volume and automatically selecting a three-dimensional spherical region of interest (ROI) within the interior of that vertebral body, the spherical ROI having a volume that is a predetermined percentage of the vertebra's volume; (e) positioning the spherical ROI such that it lies entirely within the trabecular portion of the vertebra and avoids the posterior central region of the vertebral body where a BF is located, the positioning further chosen to target a region of relatively lowest attenuation within the vertebra; (f) extracting the voxel intensities (Hounsfield Units) from the CT image within the spherical ROI and calculating a representative attenuation value for the vertebra by computing a median of said voxel intensities; (g) calibrating the representative attenuation value to a reference scale to compensate for differences in CT acquisition parameters, by applying one or more predetermined calibration factors corresponding to the CT scanner model and X-ray tube voltage used for the image volume; (h) comparing the calibrated attenuation value for the vertebra to population-based reference data stratified by age and sex; and (i) identifying a representative attenuation value which is the median Hounsfield Unit of the voxels inside the ROI, providing a robust statistic resistant to outlier high-density voxels, such that the use of the median yields a lower and more reliable trabecular attenuation measure compared to a mean value.

A method of Calibration of HU Values to enable phantom-less calibration of opportunistic BMD measurements, comprising: (a) acquiring a three-dimensional CT image volume that includes one or more vertebrae; (b) processing the CT image volume with a convolutional neural network (CNN) to automatically segment and label each vertebral body present in the volume; (c) performing consistency checks on the segmented vertebrae to ensure valid identification, including verifying that each segmented vertebral body forms a single connected component in the segmentation and that the labeled vertebrae follow a correct anatomical sequence based on their spatial arrangement; (d) for each correctly segmented vertebral body, calculating its volume and automatically selecting a three-dimensional spherical region of interest (ROI) within the interior of that vertebral body, the spherical ROI having a volume that is a predetermined percentage of the vertebra's volume; (e) positioning the spherical ROI such that it lies entirely within the trabecular portion of the vertebra and avoids the posterior central region of the vertebral body where a BF is located, the positioning further chosen to target a region of relatively lowest attenuation within the vertebra; (f) extracting the voxel intensities (Hounsfield Units) from the CT image within the spherical ROI and calculating a representative attenuation value for the vertebra by computing a median of said voxel intensities; (g) calibrating the representative attenuation value to a reference scale to compensate for differences in CT acquisition parameters, by applying one or more predetermined calibration factors corresponding to the CT scanner model and X-ray tube voltage used for the image volume; (h) comparing the calibrated attenuation value for the vertebra to population-based reference data stratified by age and sex; (i) applying a first linear scaling factor to normalize the value to an equivalent 120 kVp value and (j) applying a second factor or offset to account for the specific CT scanner model, the factors being derived from empirical analysis of bone attenuation differences across a plurality of scans acquired with different kVp settings and scanner types, thereby enabling phantom-less calibration of opportunistic BMD measurements.

A method of using Multiple Vertebrae and Median Selection to mitigate the effect of any one vertebra having atypical pathology or artifacts, further comprising: (a) acquiring a three-dimensional CT image volume that includes one or more vertebrae; (b) processing the CT image volume with a convolutional neural network (CNN) to automatically segment and label each vertebral body present in the volume; (c) performing consistency checks on the segmented vertebrae to ensure valid identification, including verifying that each segmented vertebral body forms a single connected component in the segmentation and that the labeled vertebrae follow a correct anatomical sequence based on their spatial arrangement; (d) for each correctly segmented vertebral body, calculating its volume and automatically selecting a three-dimensional spherical region of interest (ROI) within the interior of that vertebral body, the spherical ROI having a volume that is a predetermined percentage of the vertebra's volume; (e) positioning the spherical ROI such that it lies entirely within the trabecular portion of the vertebra and avoids the posterior central region of the vertebral body where a BF is located, the positioning further chosen to target a region of relatively lowest attenuation within the vertebra; (f) extracting the voxel intensities (Hounsfield Units) from the CT image within the spherical ROI and calculating a representative attenuation value for the vertebra by computing a median of said voxel intensities; (g) calibrating the representative attenuation value to a reference scale to compensate for differences in CT acquisition parameters, by applying one or more predetermined calibration factors corresponding to the CT scanner model and X-ray tube voltage used for the image volume; (h) comparing the calibrated attenuation value for the vertebra to population-based reference data stratified by age and sex; and repeating steps (d) through (g) for a plurality of vertebrae in the CT image volume, and (i) determining an overall bone mineral density measurement for the patient by aggregating the calibrated attenuation values of multiple vertebrae, where the aggregating includes selecting the median value among the vertebrae measured to represent the patient's BMD, in order to mitigate the effect of any one vertebra having atypical pathology or artifacts.

A method of using Comparison to Normative Database to automatically classify a patient's bone mineral density comprising: (a) acquiring a three-dimensional CT image volume that includes one or more vertebrae; (b) processing the CT image volume with a convolutional neural network (CNN) to automatically segment and label each vertebral body present in the volume; (c) performing consistency checks on the segmented vertebrae to ensure valid identification, including verifying that each segmented vertebral body forms a single connected component in the segmentation and that the labeled vertebrae follow a correct anatomical sequence based on their spatial arrangement; (d) for each correctly segmented vertebral body, calculating its volume and automatically selecting a three-dimensional spherical region of interest (ROI) within the interior of that vertebral body, the spherical ROI having a volume that is a predetermined percentage of the vertebra's volume; (e) positioning the spherical ROI such that it lies entirely within the trabecular portion of the vertebra and avoids the posterior central region of the vertebral body where a BF is located, the positioning further chosen to target a region of relatively lowest attenuation within the vertebra; (f) extracting the voxel intensities (Hounsfield Units) from the CT image within the spherical ROI and calculating a representative attenuation value for the vertebra by computing a median of said voxel intensities; (g) calibrating the representative attenuation value to a reference scale to compensate for differences in CT acquisition parameters, by applying one or more predetermined calibration factors corresponding to the CT scanner model and X-ray tube voltage used for the image volume; (h) comparing the calibrated attenuation value for the vertebra to population-based reference data stratified by age and sex; and (i) comparing the calibrated value to reference data includes identifying an age-specific and sex-specific percentile for the patient's measurement, and automatically classifying the patient's bone mineral density as normal, low bone mass (osteopenia), or osteoporosis by comparing the measurement to one or more threshold values corresponding to established diagnostic criteria.

A system of Deploying in PACS or Cloud to process large numbers of studies in an automated fashion comprising: one or more processors and memory storing instructions that, when executed by the one or more processors, cause the system to : (a) acquire a three-dimensional CT image volume that includes one or more vertebrae; (b) process the CT image volume with a convolutional neural network (CNN) to automatically segment and label each vertebral body present in the volume; (c) perform consistency checks on the segmented vertebrae to ensure valid identification, including verifying that each segmented vertebral body forms a single connected component in the segmentation and that the labeled vertebrae follow a correct anatomical sequence based on their spatial arrangement; (d) for each correctly segmented vertebral body, calculate its volume and automatically selecting a three-dimensional spherical region of interest (ROI) within the interior of that vertebral body, the spherical ROI having a volume that is a predetermined percentage of the vertebra's volume; (e) position the spherical ROI such that it lies entirely within the trabecular portion of the vertebra and avoids the posterior central region of the vertebral body where a BF is located, the positioning further chosen to target a region of relatively lowest attenuation within the vertebra; (f) extract the voxel intensities (Hounsfield Units) from the CT image within the spherical ROI and calculating a representative attenuation value for the vertebra by computing a median of said voxel intensities; (g) calibrate the representative attenuation value to a reference scale to compensate for differences in CT acquisition parameters, by applying one or more predetermined calibration factors corresponding to the CT scanner model and X-ray tube voltage used for the image volume; (h) compare the calibrated attenuation value for the vertebra to population-based reference data stratified by age and sex, where the system further generates a report indicating a patient's bone mineral density status based on the analysis of one or more vertebrae in an input CT scan; and (i) integrate the system with a Picture Archiving and Communication System (PACS) such that it automatically receives CT scans and returns bone mineral density analysis results to the PACS for radiologist review, or where the system is implemented as a cloud-based service accessible over a network, allowing upload of medical imaging data and returning of analysis results, with support for processing large numbers of studies in an automated fashion.

A method of using a Computer Readable Medium to determine bone mineral density in trabecular bone of vertebral bodies using CT scan data comprising: a non-transitory computer-readable medium storing program instructions that, when executed by one or more processors, cause a computer system to perform the (a) acquiring a three-dimensional CT image volume that includes one or more vertebrae; (b) processing the CT image volume with a convolutional neural network (CNN) to automatically segment and label each vertebral body present in the volume; (c) performing consistency checks on the segmented vertebrae to ensure valid identification, including verifying that each segmented vertebral body forms a single connected component in the segmentation and that the labeled vertebrae follow a correct anatomical sequence based on their spatial arrangement; (d) for each correctly segmented vertebral body, calculating its volume and automatically selecting a three-dimensional spherical region of interest (ROI) within the interior of that vertebral body, the spherical ROI having a volume that is a predetermined percentage of the vertebra's volume; (e) positioning the spherical ROI such that it lies entirely within the trabecular portion of the vertebra and avoids the posterior central region of the vertebral body where a BF is located, the positioning further chosen to target a region of relatively lowest attenuation within the vertebra; (f) extracting the voxel intensities (Hounsfield Units) from the CT image within the spherical ROI and calculating a representative attenuation value for the vertebra by computing a median of said voxel intensities; (g) calibrating the representative attenuation value to a reference scale to compensate for differences in CT acquisition parameters, by applying one or more predetermined calibration factors corresponding to the CT scanner model and X-ray tube voltage used for the image volume; and (h) comparing the calibrated attenuation value for the vertebra to population-based reference data stratified by age and sex to determine a percentile ranking or diagnostic category indicating the bone mineral density status of the vertebra for calculating vertebral trabecular bone mineral density from CT images.

A method of using a Computer Readable Medium to determine bone mineral density in trabecular bone of vertebral bodies using CT scan data comprising: (a) acquiring a three-dimensional CT image volume that includes one or more vertebrae; (b) processing the CT image volume with a convolutional neural network (CNN) to automatically segment and label each vertebral body present in the volume; (c) performing consistency checks on the segmented vertebrae to ensure valid identification, including verifying that each segmented vertebral body forms a single connected component in the segmentation and that the labeled vertebrae follow a correct anatomical sequence based on their spatial arrangement; (d) for each correctly segmented vertebral body, calculating its volume and automatically selecting a three-dimensional spherical region of interest (ROI) within the interior of that vertebral body, the spherical ROI having a volume that is a predetermined percentage of the vertebra's volume; (e) positioning the spherical ROI such that it lies entirely within the trabecular portion of the vertebra and avoids the posterior central region of the vertebral body where a BF is located, the positioning further chosen to target a region of relatively lowest attenuation within the vertebra; (f) extracting the voxel intensities (Hounsfield Units) from the CT image within the spherical ROI and calculating a representative attenuation value for the vertebra by computing a median of said voxel intensities; (g) calibrating the representative attenuation value to a reference scale to compensate for differences in CT acquisition parameters, by applying one or more predetermined calibration factors corresponding to the CT scanner model and X-ray tube voltage used for the image volume; (h) comparing the calibrated attenuation value for the vertebra to population-based reference data stratified by age and sex, where the CNN is trained to identify and label vertebral bodies across a range of spinal levels, and the CNN outputs a labeled segmentation distinguishing individual vertebrae (thoracic and/or lumbar); and (h) excluding from analysis any vertebrae that are only partially present at the edges of the CT field of view for calculating vertebral trabecular bone mineral density from CT images.

A computer system for Vertebral BMD Analysis comprising: one or more processors and memory storing instructions that, when executed by the one or more processors, cause the system to data comprising: a non-transitory computer-readable medium storing program instructions that, when executed by one or more processors, cause a computer system to perform the (a) acquiring a three-dimensional CT image volume that includes one or more vertebrae; (b) processing the CT image volume with a convolutional neural network (CNN) to automatically segment and label each vertebral body present in the volume; (c) performing consistency checks on the segmented vertebrae to ensure valid identification, including verifying that each segmented vertebral body forms a single connected component in the segmentation and that the labeled vertebrae follow a correct anatomical sequence based on their spatial arrangement; (d) for each correctly segmented vertebral body, calculating its volume and automatically selecting a three-dimensional spherical region of interest (ROI) within the interior of that vertebral body, the spherical ROI having a volume that is a predetermined percentage of the vertebra's volume; (e) positioning the spherical ROI such that it lies entirely within the trabecular portion of the vertebra and avoids the posterior central region of the vertebral body where a BF is located, the positioning further chosen to target a region of relatively lowest attenuation within the vertebra; (f) extracting the voxel intensities (Hounsfield Units) from the CT image within the spherical ROI and calculating a representative attenuation value for the vertebra by computing a median of said voxel intensities; (g) calibrating the representative attenuation value to a reference scale to compensate for differences in CT acquisition parameters, by applying one or more predetermined calibration factors corresponding to the CT scanner model and X-ray tube voltage used for the image volume; and (h) comparing the calibrated attenuation value for the vertebra to population-based reference data stratified by age and sex to determine a percentile ranking or diagnostic category indicating the bone mineral density status of the vertebra, where the system further generates a report indicating a patient's bone mineral density status based on the analysis of one or more vertebrae in an input CT scan, where the generated report includes a visual indication of the ROI placement within the vertebra on the CT images and a summary of findings including at least the calibrated HU values and corresponding bone mineral density interpretations (percentile or diagnostic category), thereby providing clinical decision support for osteoporosis screening and diagnosis.

Abbreviations used in the following embodiments and elsewhere herein are: 2D = two dimensional; 3D = three-Dimensional; CTI = Computer Tomography Image; AI = Artificial Intelligence; AIC = Artificial Intelligence Classifier; AR = Absolute Risk; AUC = Area under the Curve; BF = Basivertebral Foramen; BMD = Bone Mineral Density; CBP = Cohort Baseline Prevalence; CDF = Cumulative Distribution Function; CCDF = Complementary Cumulative Distribution Function; CNN = Convolutional Neural Network; CT = Computer Tomography; DICOM = Digital Imaging and Communication in Medicine; DXA = Dual-energy X-ray Absorptiometry; HU = Hounsfield Units; HUcalibrated = Calibrated Hounsfield Units; · HUmeasured = Measured Hounsfield Units; MRI = Magnetic Resonance Imaging; MIP = Maximum Intensity Projection; MPR = Multi Planar Reformats; NN = Neural Networks; PACS = Picture Archiving and Communication System; ROI = Region of Interest; RV = Rendered Volume; VI = Volumetric Image; VR = Volume Rendering; SegV = Segmented Vertebrae; SROI = spherical ROI; 3DIDS = three-Dimensional Image Data Set.

The present invention may be characterized by the following additional examples Q1 to Q22:.

Example Q1. A method comprising, (A) receiving a 3D CTI data set comprising a plurality of 2D (two-Dimensional) images of one or more vertebrae, (B) generating a 3D RV (Rendered Volume) from the 3D CTI data set, (C) generating one or more SegV using a CNN, where the CNN generates the one or more SegV from the 3D RV, where segmentation isolates for analysis a vertebral trabecular bone region in each of the one or more SegV, (D) calculating one or more first volumes corresponding to each of the one or more SegV, (E) calculating one or more second volumes for each of the one or more first volumes, where the one or more second volumes avoid the posterior third of the vertebral trabecular bone region in each of the one or more SegV, where the one or more second volumes avoid a basivertebral foramen in each of the one or more SegV, (F) selecting one or more spherical ROI (Regions of Interest) within the interior of the one or more second volumes, where each of the one or more spherical ROI is a 3D Volume, where the one or more spherical ROI are a predetermined percentage of the one or more first volumes, where the one or more spherical ROI are chosen to fit entirely within the one or more second volumes, where the interior of the one or more second volumes is scanned with an optimization strategy to determine a position of the one or more spherical ROI such that a median HU value is minimized, (G) extracting a plurality of voxel intensities within each of the one or more spherical ROI, (H) calculating one or more representative attenuation values for each of the plurality of voxel intensities, (I) calculating one or more calibrated attenuation values based on the one or more representative attenuation values by comparing each of the one or more representative attenuation values to a reference scale to compensate for differences in one or more acquisition parameters of the 3D CTI, and (J) comparing the one or more calibrated attenuation values to population-based reference data to determine a bone mineral density status for the one or more SegV.

Example Q2. The method of Example Q1, where, (i) one or both the 3D CTI data set and the 2D images are historical data, (ii) the CNN is trained on a first large set of manually labeled 3D CTI data sets to identify voxels that belong to vertebral bodies, (iii) the CNN is trained on a second large set of manually labeled 3D CTI data sets to recognize the vertebral trabecular bone region in one or more vertebral bodies, (iv) the CNN labels the vertebral bodies, (v) the CNN identifies a vertebral body in the one or more SegV, (vi) the CNN is used to output the one or more second volumes, (vii) the CNN is used to output the one or more spherical ROI, (viii) the CNN is used to identify in each of the one or more SegV the vertebral trabecular bone region, (ix) the CNN verifies that each of the one or more SegV comprise a single connected component; (x) the CNN verifies that each of the one or more SegV is assigned in correct anatomical sequence based on their spatial arrangement, (xi) the CNN verifies that each of the one or more SegV is correctly labelled, (xii) the one or more calibrated attenuation values are compared with a first population-based reference data stratified by age to determine a diagnostic category, (xii) the one or more calibrated attenuation values are compared with a second population-based reference data stratified by sex to determine the diagnostic category, (xiii) the one or more calibrated attenuation values are compared with a third population-based reference data stratified by both age and sex to determine the diagnostic category, (xiv) the one or more calibrated attenuation values are compared with a fourth population-based reference data to indicate the bone mineral density status of the one or more SegV, and (xv) the 3D CTI data set is received from a user and the bone mineral density status for the one or more SegV is sent to the user.

Example Q3. The method of Example Q1, where the predetermined percentage is between, a lower limit of approximately two per cent, and an upper limit of approximately eight per cent.

Example Q4. The method of Example Q1, where the one or more acquisition parameters are selected from a model of a three-dimensional Computer Tomography scanner used to generate the 3D CTI or an X-ray tube voltage used to generate the 3D CTI.

Example Q5. The method of Example Q1, further comprising generating a report indicating a patient's bone mineral density status based on analysis of the one or more SegV.

Example Q6. The method of Example Q5, where the report includes one or more of a visual indication of placement of a spherical ROI within a SegV, a calibrated average density value, and the reference scale.

Example Q7. A non-transitory computer-readable medium storing instructions that, when executed by one or more processors, perform the method of any of Examples Q1-Q6.

Example Q8. A computer system comprising, one or more processors and memory storing instructions that, when executed by the one or more processors, cause the system to perform the method of any of Examples Q1-Q6, where the computer system further generates the report indicating the bone mineral density status of the one or more SegV.

Example Q9. The computer system of Example Q8, where the report includes a visual indication of placement of a spherical ROI within the vertebra on the CTI data set and a summary of findings including at least one or more calibrated attenuation values and corresponding bone mineral density.

Example Q10. A method comprising, (A) receiving a 3D CTI data set comprising a plurality of 2D (two-Dimensional) images of one or more vertebrae, (B) generating a 3D RV (Rendered Volume) from the 3D CTI data set, (C) generating one or more SegV using a CNN, where the CNN generates the one or more SegV from the 3D RV, where the CNN is trained to identify and label vertebral bodies across a range of spinal levels, where the CNN generates a labeled segmentation distinguishing individual vertebrae, where the CNN excludes from analysis any vertebrae that are only partially present in the one or more SegV, where segmentation isolates for analysis a vertebral trabecular bone region in each of the one or more SegV, (D) calculating one or more first volumes corresponding to each of the one or more SegV, (E) calculating one or more second volumes for each of the one or more first volumes, where the one or more second volumes do not include voxels from the posterior third of the vertebral trabecular bone region of the one or more first volumes, where the one or more second volumes do not include voxels from a basivertebral foramen in each of the one or more SegV, (F) selecting one or more spherical ROI (Regions of Interest) within the interior of the one or more second volumes, where each of the one or more spherical ROI is a 3D Volume, where the one or more spherical ROI are a predetermined percentage of the one or more first volumes, where the one or more spherical ROI are chosen to fit entirely within the one or more second volumes, where the interior of the one or more second volumes is scanned with an optimization strategy to determine a position of the one or more spherical ROI such that a median HU value is minimized, (G) extracting a plurality of voxel intensities within each of the one or more spherical ROI, (H) calculating one or more representative attenuation values for each of the plurality of voxel intensities, (I) calculating one or more calibrated attenuation values based on the one or more representative attenuation values by comparing each of the one or more representative attenuation values to a reference scale to compensate for differences in one or more acquisition parameters of the 3D CTI, and (J) comparing the one or more calibrated attenuation values to population-based reference data to determine a bone mineral density status for the one or more SegV.

Example Q11. The method of Example Q10, where, (i) one or both the 3D CTI data set and the 2D images are historical data, (ii) the CNN is trained on a first large set of manually labeled 3D CTI data sets to identify voxels that belong to vertebral bodies, (iii) the CNN is trained on a second large set of manually labeled 3D CTI data sets to recognize the vertebral trabecular bone region in one or more vertebral bodies, (iv) the CNN labels the vertebral bodies, (v) the CNN identifies a vertebral body in the one or more SegV, (vi) the CNN is used to output the one or more second volumes, (vii) the CNN is used to output the one or more spherical ROI, (viii) the CNN is used to identify in each of the one or more SegV the vertebral trabecular bone region, (ix) the CNN verifies that each of the one or more SegV comprise a single connected component; (x) the CNN verifies that each of the one or more SegV is assigned in correct anatomical sequence based on their spatial arrangement, (xi) the CNN verifies that each of the one or more SegV is correctly labelled, (xii) the one or more calibrated attenuation values are compared with a first population-based reference data stratified by age to determine a diagnostic category, (xii) the one or more calibrated attenuation values are compared with a second population-based reference data stratified by sex to determine the diagnostic category, (xiii) the one or more calibrated attenuation values are compared with a third population-based reference data stratified by both age and sex to determine the diagnostic category, (xiv) the one or more calibrated attenuation values are compared with a fourth population-based reference data to indicate the bone mineral density status of the one or more SegV, and (xv) the 3D CTI data set is received from a user and the bone mineral density status for the one or more SegV is sent to the user.

Example Q12. The method of Example Q10, where the predetermined percentage is between, a lower limit of approximately three per cent, and an upper limit of approximately seven per cent.

Example Q13. The method of Example Q10, where the one or more representative attenuation values are calculated by computing a median of the plurality of voxel intensities.

Example Q14. The method of Example Q10, further comprising generating a report indicating a patient's bone mineral density status based on analysis of the one or more SegV.

Example Q15. The method of Example Q14, where the report includes one or more of a visual indication of ROI placement within a SegV, a calibrated average density value, and the reference scale.

Example Q16. A non-transitory computer-readable medium storing instructions that, when executed by one or more processors, perform the method of any of Examples Q10-Q15.

Example Q17. A computer system comprising, one or more processors and memory storing instructions that, when executed by the one or more processors, cause the system to perform the method of any of Examples Q10-Q15, where the computer system further generates a report indicating a bone mineral density status of the one or more SegV.

Example Q18. A computer system for vertebral bone mineral density analysis comprising, one or more processors and memory storing instructions that, when executed by the one or more processors, cause the computer system to determine a bone mineral density status for one or more SegV comprising, a non-transitory computer-readable medium storing program instructions that when executed by the one or more processors, cause the computer system to perform steps comprising, (A) receive a 3D CTI data set comprising a plurality of two-Dimensional images of one or more vertebrae, (B) generate one or more 3D RVs (Rendered Volumes) from the 3D CTI data set, (C) generate the one or more SegV using a CNN, where the CNN generates the one or more SegV from the 3D RV, (D) identify one or more vertebral trabecular bone regions in each of the one or more SegV, (E) calculate one or more first volumes corresponding to each of the one or more SegV, (F) calculate one or more second volumes for each of the one or more first volumes, where the one or more second volumes do not include voxels from the posterior third of the one or more vertebral trabecular bone regions of the one or more first volumes, where the one or more second volumes do not include voxels from a basivertebral foramen in each of the one or more SegV, (G) select one or more spherical ROI (Regions of Interest) within the interior of the one or more second volumes, where each of the one or more spherical ROI is a 3D Volume, where the one or more spherical ROI are a predetermined percentage of the one or more first volumes, where the one or more spherical ROI are chosen to fit entirely within the one or more second volumes, where the interior of the one or more second volumes is scanned with an optimization strategy to determine a position of the one or more spherical ROI such that a median HU value is minimized, (H) extract a plurality of voxel intensities within each of the one or more spherical ROI, (I) calculate one or more representative attenuation values for each of the plurality of voxel intensities, (J) calculate one or more calibrated attenuation values based on the one or more representative attenuation values by comparing each of the one or more representative attenuation values to a reference scale to compensate for differences in one or more acquisition parameters of the 3D CTI, and (K) compare the one or more calibrated attenuation values to population-based reference data to determine the bone mineral density status for the one or more SegV.

Example Q19. The computer system of Example Q18, where, (i) one or both the 3D CTI data set and the 2D images are historical data, (ii) the CNN is trained on a large set of manually labeled 3D CTIs to recognize vertebral bodies and differentiate from other structures, (iii) the CNN labels the vertebral bodies in each 3D CTI in the data set, (iv) the CNN is used to identify the one or more SegV as either a thoracic vertebral body or a lumbar vertebral body in the 3D CTI, (v) the CNN is used to label the one or more SegV in the 3D CTI, (vi) the CNN is used to isolate a vertebral trabecular bone in each of the one or more SegV, (vii) the CNN is used on a 3D CTI data set to output a segmentation mask identifying voxels that belong to bone, (viii) the CNN is used to identify in each of the one or more SegV the vertebral trabecular bone region, (ix) the CNN verifies that each of the one or more SegV comprise a single connected component; (x) the CNN verifies that each of the one or more SegV is assigned in correct anatomical sequence based on their spatial arrangement, (xi) the CNN verifies that each of the one or more SegV is correctly labelled, (xii) the one or more calibrated attenuation values are compared with a first population-based reference data stratified by age to determine a diagnostic category, (xii) the one or more calibrated attenuation values are compared with a second population-based reference data stratified by sex to determine the diagnostic category, (xiii) the one or more calibrated attenuation values are compared with a third population-based reference data stratified by both age and sex to determine the diagnostic category, (xiv) the one or more calibrated attenuation values are compared with a fourth population-based reference data to indicate the bone mineral density status of the one or more SegV, and (xv) the 3D CTI data set is received from a user and the bone mineral density status for the one or more SegV is sent to the user.

Example Q20. The computer system of Example Q18, where the computer system further generates a report indicating a patient's bone mineral density status based on analysis of the one or more SegV.

Example Q21. The computer system of Example Q20, where the report includes one or more of a visual indication of ROI placement within the one or more SegV, a calibrated average density value, and the reference scale.

Example Q22. The computer system of Example Q18, where the one or more representative attenuation values are calculated by computing a median of voxel intensities.

The reference to any prior art in this specification is not, and should not be taken as, an acknowledgement, admission, or any form of suggestion that the prior art forms part of the common general knowledge.

## Claims

1. A method comprising:
(A) receiving a 3D (three-Dimensional) CTI (Computer Tomography Image) data set comprising a plurality of 2D (two-Dimensional) images of one or more vertebrae;
(B) generating a 3D RV (Rendered Volume) from the 3D CTI data set;
(C) generating one or more SegV (Segmented Vertebrae) using a CNN (Convolutional Neural Network), where the CNN generates the one or more SegV from the 3D RV, where segmentation isolates for analysis a vertebral trabecular bone region in each of the one or more SegV;
(D) calculating one or more first volumes corresponding to each of the one or more SegV;
(E) calculating one or more second volumes for each of the one or more first volumes, where the one or more second volumes do not include voxels from the posterior third of the vertebral trabecular bone region in each of the one or more first volumes, where the one or more second volumes do not include voxels from a basivertebral foramen in each of the one or more first volumes;
(F) selecting one or more spherical ROI (Regions of Interest) within the interior of the one or more second volumes, where each of the one or more spherical ROI is a 3D Volume, where the one or more spherical ROI are a predetermined percentage of the one or more first volumes, where the one or more spherical ROI are chosen to fit entirely within the one or more second volumes, where the interior of the one or more second volumes is scanned with an optimization strategy to determine a position of the one or more spherical ROI such that a median HU value is minimized;
(G) extracting a plurality of voxel intensities within each of the one or more spherical ROI;
(H) calculating one or more representative attenuation values for each of the plurality of voxel intensities;
(I) calculating one or more calibrated attenuation values based on the one or more representative attenuation values by comparing each of the one or more representative attenuation values to a reference scale to compensate for differences in one or more acquisition parameters of the 3D CTI; and
(J) comparing the one or more calibrated attenuation values to population-based reference data to determine a bone mineral density status for the one or more SegV.

2. The method of claim 1, where:
(i) one or both the 3D CTI data set and the 2D images are historical data;
(ii) the CNN is trained on a first large set of manually labeled 3D CTI data sets to identify voxels that belong to vertebral bodies;
(iii) the CNN is trained on a second large set of manually labeled 3D CTI data sets to recognize the vertebral trabecular bone region in one or more vertebral bodies;
(iv) the CNN labels the vertebral bodies;
(v) the CNN identifies a vertebral body in the one or more SegV;
(vi) the CNN is used to output the one or more second volumes;
(vii) the CNN is used to output the one or more spherical ROI;
(viii) the CNN is used to identify in each of the one or more SegV the vertebral trabecular bone region;
(ix) the CNN verifies that each of the one or more SegV comprise a single connected component;
(x) the CNN verifies that each of the one or more SegV is assigned in correct anatomical sequence based on their spatial arrangement;
(xi) the CNN verifies that each of the one or more SegV is correctly labelled;
(xii) the one or more calibrated attenuation values are compared with a first population-based reference data stratified by age to determine a diagnostic category;
(xii) the one or more calibrated attenuation values are compared with a second population-based reference data stratified by sex to determine the diagnostic category;
(xiii) the one or more calibrated attenuation values are compared with a third population-based reference data stratified by both age and sex to determine the diagnostic category;
(xiv) the one or more calibrated attenuation values are compared with a fourth population-based reference data to indicate the bone mineral density status of the one or more SegV; and
(xv) the 3D CTI data set is received from a user and the bone mineral density status for the one or more SegV is sent to the user.

3. The method of claim 1, where the predetermined percentage is between:
a lower limit of approximately two per cent; and
an upper limit of approximately eight per cent.

4. The method of claim 1, where the one or more acquisition parameters are selected from a model of a three-dimensional Computer Tomography scanner used to generate the 3D CTI or an X-ray tube voltage used to generate the 3D CTI.

5. The method of claim 1, further comprising generating a report indicating a patient's bone mineral density status based on analysis of the one or more SegV.

6. The method of claim 5, where the report includes one or more of a visual indication of placement of a spherical ROI within a SegV of the one or more SegV, a calibrated average density value, and the reference scale.

7. A non-transitory computer-readable medium storing instructions that, when executed by one or more processors, perform the method of any of claims 1-6.

8. A computer system comprising:
one or more processors and memory storing instructions that, when executed by the one or more processors, cause the computer system to perform the method of any of claims 1-6, where the computer system further generates the report indicating the bone mineral density status of the one or more SegV.

9. The computer system of claim 8, where the report includes a visual indication of placement of a spherical ROI within a SegV of the one or more SegV on the CTI data set and a summary of findings including at least one or more calibrated attenuation values and corresponding bone mineral density.

10. A method comprising:
(A) receiving a 3D (three-Dimensional) CTI (Computer Tomography Image) data set comprising a plurality of 2D (two-Dimensional) images of one or more vertebrae;
(B) generating a 3D RV (Rendered Volume) from the 3D CTI data set;
(C) generating one or more SegV (Segmented Vertebrae) using a CNN (Convolutional Neural Network), where the CNN generates the one or more SegV from the 3D RV, where the CNN is trained to identify and label vertebral bodies across a range of spinal levels, where the CNN generates a labeled segmentation distinguishing individual vertebrae, where the CNN excludes from analysis any vertebrae that are only partially present in the one or more SegV, where segmentation isolates for analysis a vertebral trabecular bone region in each of the one or more SegV;
(D) calculating one or more first volumes corresponding to each of the one or more SegV;
(E) calculating one or more second volumes for each of the one or more first volumes, where the one or more second volumes do not include voxels from the posterior third of the vertebral trabecular bone region of the one or more first volumes, where the one or more second volumes do not include voxels from a basivertebral foramen in each of the one or more first volumes;
(F) selecting one or more spherical ROI (Regions of Interest) within the interior of the one or more second volumes, where each of the one or more spherical ROI is a 3D Volume, where the one or more spherical ROI are a predetermined percentage of the one or more first volumes, where the one or more spherical ROI are chosen to fit entirely within the one or more second volumes, where the interior of the one or more second volumes is scanned with an optimization strategy to determine a position of the one or more spherical ROI such that a median HU value is minimized;
(G) extracting a plurality of voxel intensities within each of the one or more spherical ROI;
(H) calculating one or more representative attenuation values for each of the plurality of voxel intensities;
(I) calculating one or more calibrated attenuation values based on the one or more representative attenuation values by comparing each of the one or more representative attenuation values to a reference scale to compensate for differences in one or more acquisition parameters of the 3D CTI; and
(J) comparing the one or more calibrated attenuation values to population-based reference data to determine a bone mineral density status for the one or more SegV.

11. The method of claim 10, where:
(i) one or both the 3D CTI data set and the 2D images are historical data;
(ii) the CNN is trained on a first large set of manually labeled 3D CTI data sets to identify voxels that belong to the vertebral bodies;
(iii) the CNN is trained on a second large set of manually labeled 3D CTI data sets to recognize the vertebral trabecular bone region in one or more vertebral bodies;
(iv) the CNN labels the vertebral bodies;
(v) the CNN identifies a vertebral body in the one or more SegV;
(vi) the CNN is used to output the one or more second volumes;
(vii) the CNN is used to output the one or more spherical ROI;
(viii) the CNN is used to identify in each of the one or more SegV the vertebral trabecular bone region;
(ix) the CNN verifies that each of the one or more SegV comprise a single connected component;
(x) the CNN verifies that each of the one or more SegV is assigned in correct anatomical sequence based on their spatial arrangement;
(xi) the CNN verifies that each of the one or more SegV is correctly labelled;
(xii) the one or more calibrated attenuation values are compared with a first population-based reference data stratified by age to determine a diagnostic category;
(xii) the one or more calibrated attenuation values are compared with a second population-based reference data stratified by sex to determine the diagnostic category;
(xiii) the one or more calibrated attenuation values are compared with a third population-based reference data stratified by both age and sex to determine the diagnostic category;
(xiv) the one or more calibrated attenuation values are compared with a fourth population-based reference data to indicate the bone mineral density status of the one or more SegV; and
(xv) the 3D CTI data set is received from a user and the bone mineral density status for the one or more SegV is sent to the user.

12. The method of claim 10, where the predetermined percentage is between:
a lower limit of approximately three per cent; and
an upper limit of approximately seven per cent.

13. The method of claim 10, where the one or more representative attenuation values are calculated by computing a median of the plurality of voxel intensities.

14. The method of claim 10, further comprising generating a report indicating a patient's bone mineral density status based on analysis of the one or more SegV.

15. The method of claim 14, where the report includes one or more of a visual indication of ROI placement within a SegV, a calibrated average density value, and the reference scale.
